# EUROPEAN PATENT SPECIFICATION

(11) **EP 1 862 473 B1**
(45) Date of publication and mention of the grant of the patent: **06.05.2009**
(21) Application number: 07075398.3
(22) Date of filing: 28.07.2003
(51) Int. Cl.: C07K 14/415, C12N 15/82, C12N 15/31

(54) **Corn root preferential promoters and uses thereof**
Maiswurzel-spezifische Promotoren und ihre Verwendungen
Promoteurs préférentiels des racines du maïs et utilisations associées

(30) Priority: 31.07.2002 US 399383 P
(43) Date of publication of application: 05.12.2007
(62) Divisional of application: 03766342.4
(73) Proprietor: Bayer BioScience N.V., 9052 Gent (BE)
(72) Inventor: Vanderkimpen, Greet, 9040 Sint-Amandsberg (BE); Van Eldik, Gerben, 9052 Zwijnaarde (BE); Meulewaeter, Frank, 9820 Merelbeke (BE)

(56) References cited:
- WO-A-00/53763
- DATABASE EM-GSS 22 February 2002 (2002-02-22), WALBOT V.: "Maize genomic sequence found using engineered RescueMu transposon" XP002457191 Database accession no. BH637179

## Description

### Field of the invention.

The invention relates to the isolation of promoters from corn capable of directing transcription of an operably linked foreign DNA sequence preferentially, selectively or exclusively in the roots of plants, such as corn plants. The invention also relates to the use of chimeric genes for the preferential or selective expression of biologically active RNA of interest in the roots of plants, such as corn plants. Plants, such as corn plants, comprising corn root preferential or selective promoters operably linked to a foreign DNA sequence which upon transcription yield biologically active RNA preferentially or selectively in the roots of plants are also provided.

### Description of related art.

A significant consideration in the production of transgenic plants is obtaining sufficient levels of expression of the transgene in the tissues of interest in a preferential or selective way. In this way, potential drawbacks associated with the constitutive expression of the transcript, such as yield drag, may be avoided. Selection of appropriate promoters is crucial for obtaining the pattern of expression of interest with a particular transgene.

Selective expression of transgenes in roots of plants, particularly cereal plants, such as corn, is considered to be potentially commercially important, e.g. for alteration of the function of root tissue, resistance to pathogens or pests with a preference for attack of roots (such as nematodes, corn rootworm etc.), resistance to herbicides or adverse environmental conditions (such as drought or soil composition).

US 5,633,363 describes a 4.7 kb upstream promoter region designated ZRP2 isolated from maize and attributes a particular utility to this promoter region in driving root preferential expression of heterologous genes.

WO 97/44448 relates generally to mechanisms of gene expression in plants and more specifically to regulation of expression of genes in plants in a tissue-specific manner particularly in roots. A method for isolation of transcriptional regulatory elements that contribute to tissue-preferred gene expression is disclosed.

WO 00/15662 describes a promoter of a glycine rich protein (zmGRP3) whose transcripts accumulate exclusively in roots of young maize seedlings following developmentally specific patterns.

WO 00/070068 and WO 00/70066 describe respectively the maize RS81 and RS324 promoters which are promoters of genes expressed in maize root tissue but not in kernel tissue and in molecular analysis were described to have a root-specific expression profile.

Despite the fact that corn root preferential promoters are available in the art, a need remains for alternative promoters capable of preferential or selective root selective expression, e.g. for the independent expression of several foreign DNA sequences of interest without the possibility of posttranscriptional silencing due to the use of the same promoter. In addition, the known corn root preferential promoters, each direct a particular temporal, spatial and/or developmental expression pattern, which does not always suit particular goals. There remains thus a need for novel corn root preferential promoters with the capacity to control transcription in roots, preferably in a more selective manner, and also preferably resulting in a highly abundant transcription product.

### Summary and objects of the invention.

It is an object of the invention to provide corn root preferential promoters comprising the nucleotide sequence of SEQ ID No 14 from the nucleotide at position 1 to the nucleotide at position 1280 ("GL5 promoter"), or a nucleotide sequence having at least 95% sequence identity therewith.

The corn root preferential promoters may be comprised within a corn root preferential promoter region, and may further comprise the nucleotide sequence of (SEQ IQ NO 1 from the nucleotide at position 339 to the nucleotide at position 366 or the nucleotide sequence of SEQ ID NO 14 from the nucleotide at position 1281 to the nucleotide at position 1308.

It is another object of the invention to provide chimeric genes comprising the following operably linked DNA regions: a corn root preferential promoter according to the invention; a heterologous DNA region encoding a biologically active RNA of interest; and a transcription termination and polyadenylation signal active in plant cells. The biologically active RNA may encode a protein of interest, such as a protein which when expressed in the cells of a plant confers pest or pathogen resistance to said plant. The biologically active RNA may also be an antisense, sense or double stranded RNA useful for post transcriptional silencing of a target gene of interest.

Also provided are plant cells and plants or seeds thereof, particularly cereal plants, such as corn plants comprising a chimeric gene according to the invention.

It is yet another objective to provide a method for expressing a biologically active RNA preferentially in the roots of a plant, such as a corn plant, comprising the steps of: providing the cells of the roots of said plants with a chimeric gene according to the invention; and growing the plants.

The invention further provides the use of a corn root preferential promoter according to the invention for preferential expression of a biologically active RNA in roots of a plant, such as a corn plant.

### Brief description of the figures.

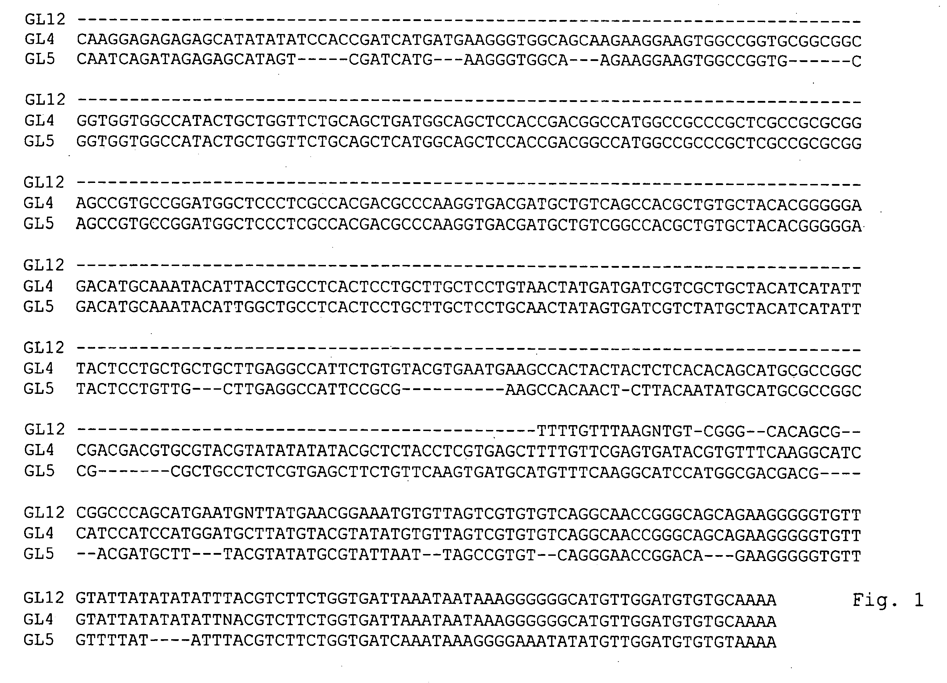

Figure 1: Alignment of the nucleotide sequences for cDNAs GL4, GL5 and GL12. Gaps in the sequence introduced for optimal alignment are indicated by a dash.

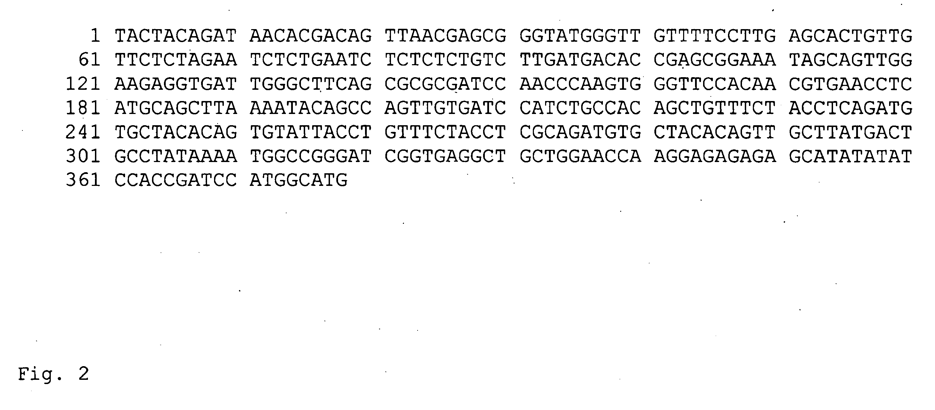

Figure 2: Nucleotide sequence for the short corn root preferential promoter region from GL4.

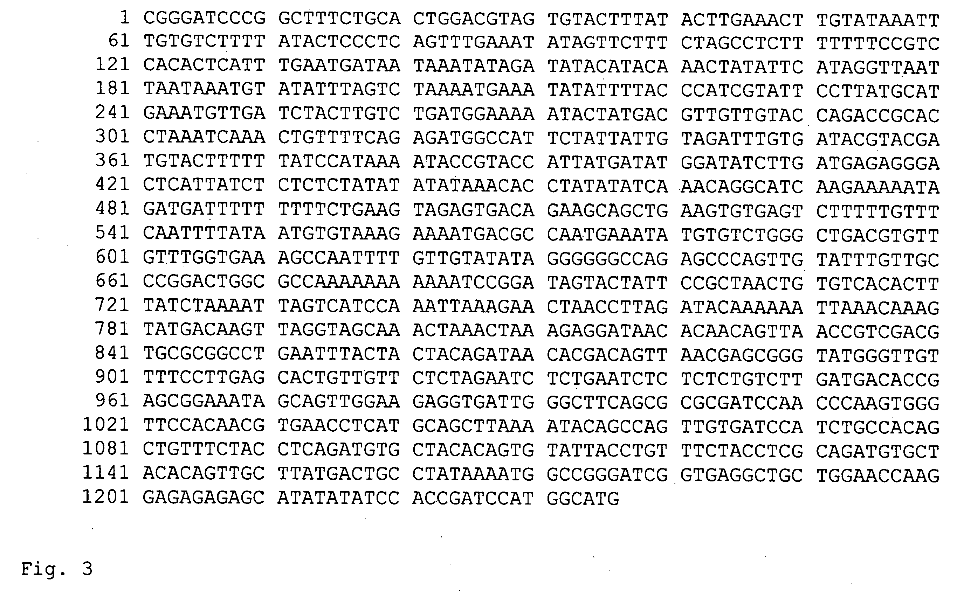

Figure 3: Nucleotide sequence for the long corn root preferential promoter region from GL4.

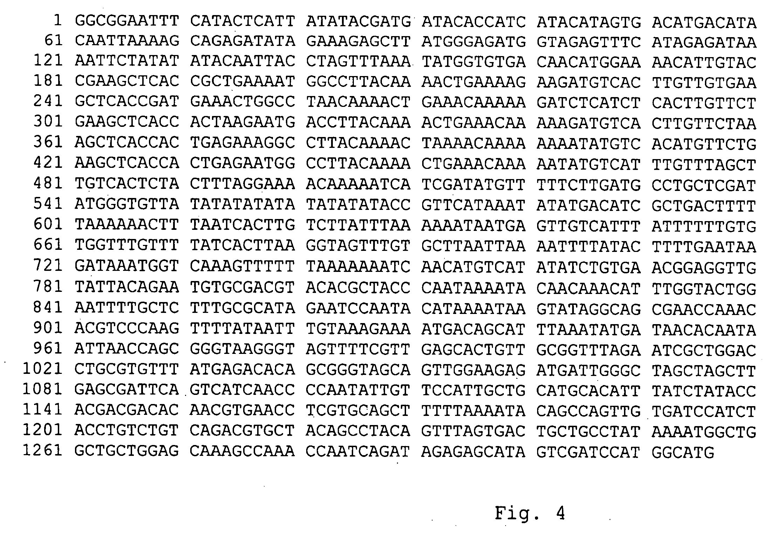

Figure 4: Nucleotide sequence for the corn root preferential promoter region from GL5.

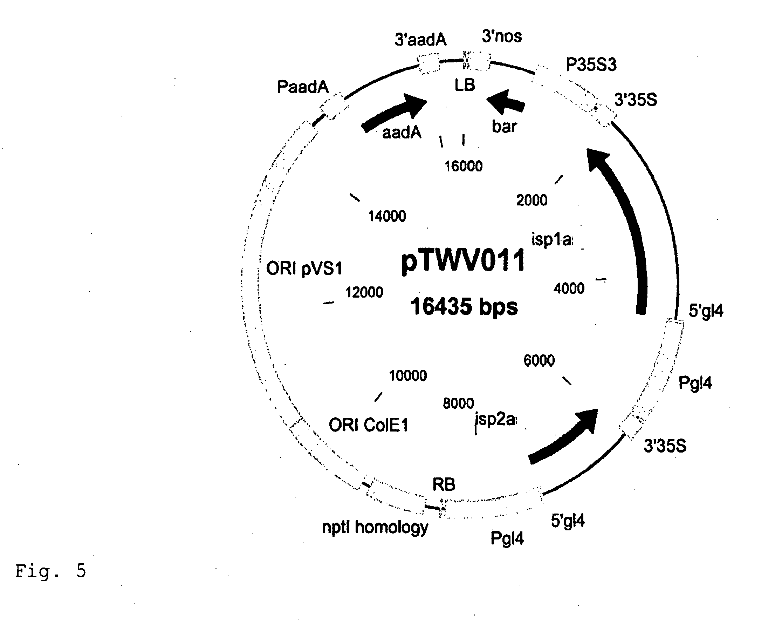

Figure 5: Schematic representation of pTWV011. LB: left T-DNA border; 3'nos: 3' end of the nopaline synthase gene; bar: bialaphos resistance coding region; P35S3: promote region of the 35S transcript of CaMV; 3' 35S: 3' end of the 35S transcript of CaMV; isp1a: coding region for insecticial secreted protein 1a from *Brevibacillus laterosporus:* 5'gl4: leader region of the GL4 promoter region; Pgl4: corn root selective promoter GL4; isp2a: coding region for insecticial secreted protein 2a from *Brevibacillus laterosporus;* RB: right T-DNA border region; nptl homology: region of homology with helper Ti-plasmids; ORI coIE1: coIE1 origin of replication; ORI pVS1: origin of replication for Pseudomonas; PaadA: bacterial promoter of the aminoglycoside adenyltransferase conferring resistance to streptomycin and spectinomycin; aadA: coding region of the aminoglycoside adenyltransferase gene; 3' aadA: 3' end of the aminoglycoside adenyltransferase gene.

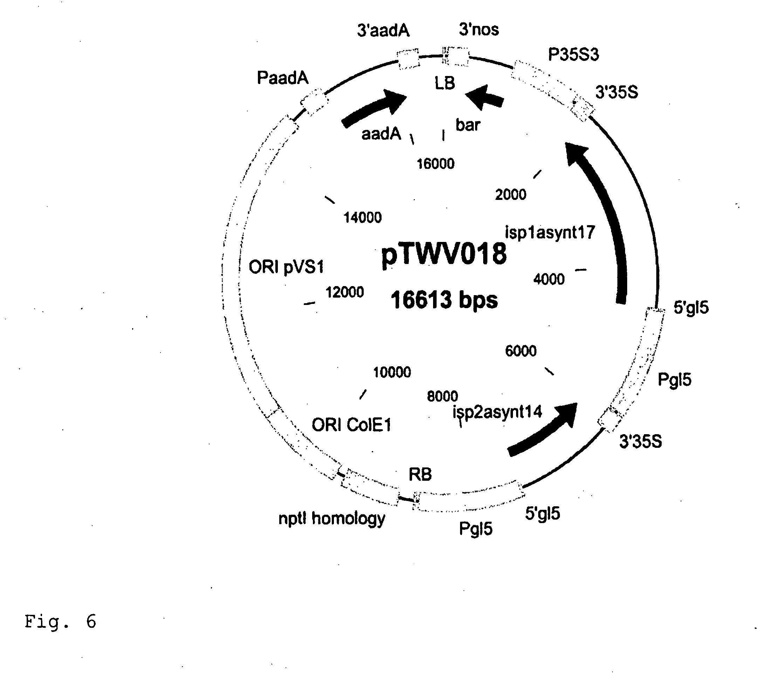

Figure 6: Schematic representation of pTWV018. LB: left T-DNA border; 3'nos: 3' end of the nopaline synthase gene; bar: blalaphos resistance coding region; P35S3:, promoter region of the 35S transcript of CaMV; 3' 35S: 3' end of the 35S transcript of CaMV; isp1a: coding region for Insecticial secreted protein 1a from *Brevibacillus laterosporus;* 5'g15: leader region of the GL5 promoter region; PgI5: corn root selective promoter GL5; isp2a: coding region for insecticial secreted protein 2a from *Brevibacillus laterosporus;* RB: right T-DNA border region; nptl homology: region of homology with helper Ti-plasmids; ORI coIE1: coIE1 origin of replication; ORI pVS1: origin of replication for Pseudomonas; PaadA: bacterial promoter of the aminoglycoside adenyltransferase conferring resistance to streptomycin and spectinomycin; aadA: coding region of the aminoglycoside adenyltransferase gene; 3' aadA: '3' end of the aminoglycoside adenyltransferase gene.

### Detailed description of preferred embodiments.

The invention is based on the finding that the promoters described herein are particularly suited for the preferential and abundant expression (i.e. transcription or transcription and translation) of an operably linked foreign DNA in roots of plants, particularly cereal plants such as corn.

In one embodiment of the invention, a com root preferential promoter region is provided comprising the nucleotide sequence of SEQ ID No 14 from the nucleotide at position 1 to the nucleotide at position 1280.

As used herein "corn" refers to maize i.e. *Zea mays L.*

As used herein, the term "promoter" denotes any DNA which is recognized and bound (directly or indirectly) by a DNA-dependent RNA-polymerase during initiation of transcription. A promoter includes the transcription initiation site, and binding sites for transcription initiation factors and RNA polymerase, and can comprise various other sites (e.g., enhancers), at which gene expression regulatory proteins may bind.

The term "regulatory region", as used herein, means any DNA, that is involved in driving transcription and controlling (i.e., regulating) the timing and level of transcription of a given DNA sequence, such as a DNA coding for a protein or polypeptide. For example, a 5' regulatory region (or "promoter region") is a DNA sequence located upstream (i.e., 5') of a coding sequence and which comprises the promoter and the 5'-untranslated leader sequence. A 3' regulatory region is a DNA sequence located downstream (i.e., 3') of the coding sequence and which comprises suitable transcription 3' end formation (and/or regulation) signals, including one or more polyadenylation signals.

The term "gene" means any DNA fragment comprising a DNA region (the "transcribed DNA region") that is transcribed into a RNA molecule (e.g., a mRNA) in a cell under control of suitable regulatory regions, e.g., a plant expressible promoter region. A gene may thus comprise several operably linked DNA fragments such as a promoter, a 5' untranslated leader- sequence, a coding region, and a 3' untranslated region comprising a polyadenylation site. An endogenous plant gene is a gene which is naturally found in a plant species. A chimeric gene is any gene which is not normally found in a plant species or, alternatively, any gene in which the promoter is not associated in nature with part or all of the transcribed DNA region or with at least one other regulatory regions of the gene.

The term "expression of a gene" refers to the process wherein a DNA region under control of regulatory regions, particularly the promoter, is transcribed into an RNA which is biologically active i.e., which is either capable of interaction with another nucleic acid or which is capable of being translated into a biologically active polypeptide or protein. A gene is said to encode an RNA when the end product of the expression of the gene is biologically active RNA; such as an antisense RNA or a ribozyme. A gene is said to encode a protein when the end product of the expression of the gene is a biologically active protein or polypeptide.

The term "root-selective", with respect to the expression of a DNA in accordance with this invention, refers to, for practical purposes, the highly specific, expression of a DNA in cells of roots of plants, such as corn plants ("corn-root-selective"). In other words, transcript levels of a DNA in tissues different of root plants is either below detection or very low (less than about 0.2 pico grammes per microgram total RNA).

The term "root-preferential" with respect to the expression of a DNA in accordance with this invention, refers to an expression pattern whereby the DNA is expressed predominantly in roots, but expression can be identified in other tissues of the plant. Preferably, the expression in roots is about 2 to about 10 times higher in roots than in other tissues.

It will be clear that having read these embodiments, the person skilled in the art can easily identify and use functional equivalent promoters for the same purposes.

DNA sequences which have a promoter activity substantially similar to the corn root preferential promoters comprising the nucleotide sequence of SEQ ID NO 14 from the nucleotide at position 1 to the nucleotide at position 1280, or parts thereof having promoter activity, are similar to this promoters. These similar promoters may hybridize with the corn root preferential promoter regions comprising the nucleotide sequence of SEQ ID NO 14 under stringent hybridization conditions.

"Stringent hybridization conditions" as used herein means that hybridization will generally occur if there is at least 95% and preferably at least 97% sequence identity between the probe and the target sequence. Examples of stringent hybridization conditions are overnight Incubation in a solution comprising 50% formamide, 5 x SSC (150 mM NaCl, 15 mM trisodium citrate), 50 mM sodium phosphate (pH 7.6), 5x Denhardt's solution, 10% dextran sulfate, and 20 µg/ml denatured, sheared carrier DNA such as salmon sperm DNA, followed by washing the hybridization support in 0.1 x SSC at approximately 65°C: Other hybridization and wash conditions are well known and are exemplified in Sambrook et al, Molecular Cloning: A Laboratory Manual, Second Edition, Cold Spring Harbor, NY (1989); particularly chapter 11.

Similar promoters may be isolated e.g. from different corn varieties. They may also be made by modifying isolated corn root preferential promoters through addition, substitution, deletion or insertion of nucleotides. They can also be completely or partly synthesized.

Alternatively, similar promoters may be isolated using a cDNA of a transcript which is expressed at a high level in roots of a plant, such as a corn plant, as a probe to isolate the genomic DNA upstream of the nucleotide sequence corresponding to the nucleotide sequence of the cDNA. As used herein "cDNA" is used to indicate both the first strand cDNA (complementary to the mRNA) as well as the strand complementary thereto (and thus identical to the mRNA except that U is replaced by T) or a double stranded cDNA fragment. Corn root selective cDNAs and their corresponding plant genomic DNA fragments may be identified as follows:
1) a c.DNA library may be constructed starting from mRNA isolated from roots and the cDNA library subjected to differential screening in order to identify an mRNA which is preferentially present in roots when compared to other plant tissues including but not limited to: leaves, seeds, stems, reproductive organs, and the like. Alternatively, the cDNA library may be screened with oligonucleotides that are deduced from a determined amino acid sequence of an isolated protein that has been identified to be preferentially present in the roots. Furthermore, it is possible to use the same oligonucleotides in a nested-PCR approach and to use the amplified fragment(s) as a probe to screen the library. The scorn root preferential cDNA library can be constructed from a pool of mRNA, isolated at different stages of corn root development. One method to identify and isolate the 3' ends of cDNA of RNA particularly expressed in a specific tissue such as here the roots of plants, is the so-called READS analysis or Restriction-Enzyme digested cDNAs as described e.g. by Prashar and Weismann or US patent 5712126.
2) a cDNA reverse transcribed from RNA preferentially transcribed in roots of plants, such as corn plants, or 3' ends of cDNAs identified by READS differential display analysis as expressed preferentially in roots of plants may be isolated and further characterized by e.g. nucleotide sequence determination; a full length cDNA may be isolated using e.g. 5' RACE (rapid amplification of cDNA ends) technology.
3) this cDNA or the 3' end thereof may be used as a probe to identify and isolate the region in the plant genome, comprising the nucleotide sequence encoding the corn root preferential mRNA. Alternatively, the genomic DNA can be isolated by e.g. inverse PCR using oligonucleotides deduced from the cDNA sequence. Alternatively, TAIL-PCR (thermal assymetric interlaced PCR as described by Liu et al. (1995)) using nested long specific oligonucleotides derived from the nucleotide sequence of the (5' end) of the identified cDNA and a short arbitrary degenerate primer may be used to isolate the genomic sequences flanking the coding region.
4) optionally, RNA probes corresponding to the cDNAs are constructed and used in conventional RNA-RNA in-situ hybridization analysis [see e.g., De Block et al. (1993), Anal. Biochem. 215: 86] of different plant tissues, including the root tissue of interest, to confirm the preferential presence of the mRNA produced by the endogenous plant gene presumed root preferential expression in those roots.

Once the corn root-prefential gene (i.e., the genomic DNA fragment, encoding the corn root-preferential mRNA from which the corn-root preferential cDNA can be prepared) is obtained, the promoter region containing the corn root - preferential promoter is determined as the region upstream (i.e., located 5' of) rom the codon coding for the first amino acid of the protein encoded by the mRNA. It is preferred that such promoter region is at least about 400 to 500 bp, preferably at least about 1000 bp, more preferably about 1200 bp, particularly about 1300 bp, especially, at least about 1500 to 2000 bp, upstream of the start codon. For convenience, it is preferred that such promoter region does not extend more than about 3000 to 5000 bp upstream of the start codon. The size fragment may be partially determined by the presence of convenient restriction sites. The actual corn root-preferential promoter is the region of the genomic DNA upstream (i.e., 5') of the region encoding the corn root-preferential mRNA. A chimeric gene comprising a corn root-preferential promoter operably linked to the coding region of a marker gene will produce the marker protein preferentially in the cells of the corn roots of transgenic corn plants, which can be assayed by conventional in-situ histochemical techniques.

Such a corn root-preferential gene is the gene that encodes a corn root-preferential mRNA from which a cDNA can be prepared that contains the sequence of SEQ ID No 5, or that contains a nucleotide sequence encoding the protein comprising the amino acid sequence of SEQ ID No 6.

Artificial promoters can be constructed which contain those internal portions of the promoter of the 5' regulatory region of SEQ ID No 1 or SEQ ID No 2 or SEQ ID NO 14 that determine the corn root-preference of this promoter. These artificial promoters might contain a "core promoter" or "TATA box region" of another promoter capable of expression in plants, such as a CaMV 35S "TATA box region" as described in WO 93/19188. The suitability of promoter regions containing such artificial promoters may be identified by their appropriate fusion to a reporter gene and the detection of the expression of the reporter gene in the appropriate tissue(s) and at the appropriate developmental stage. It is believed that such smaller promoters and/or artificial promoters comprising those internal portions of the 5' regulatory region of SEQ ID No. 1 or 2 that determine the corn root preference can provide better selectivity of transcription in corn root cells and/or provide enhanced levels of transcription of the transcribed regions of the corn root-preferential chimeric genes described herein. Such smaller portions of the corn root preferential promoter regions described herein may include the nucleotide sequences which share a high homology between the GL4 and GL5 promoter regions such as: the nucleotide sequence of SEQ ID No 2 from the nucleotide at position 1024 to the nucleotide at position 1105 (having a 80 % match with the nucleotide sequence of SEQ ID No 14 from the nucleotide at position 435 to the nucleotide at position 510); the nucleotide sequence of SEQ ID No 2 from the nucleotide at position 866 to the nucleotide at position 994 (having a 77 % match with the nucleotide sequence of SEQ ID No 14 from the nucleotide at position 236 to the nucleotide at position 358); the nucleotide sequence of SEQ ID No 2 from the nucleotide at position 544 to the nucleotide at position 568 (having a 96% match with the nucleotide sequence of SEQ ID No 15 from the nucleotide at position 198 to the nucleotide at position 222); the nucleotide sequence of SEQ ID No 2 from the nucleotide at position 1122 to the nucleotide at position 1143 (having a 73 % match with the nucleotide sequence of SEQ ID No 15 from the nucleotide at position 485 to the nucleotide at position 510).

Besides the actual promoter, the 5' regulatory region of the corn root-preferential genes of this invention also comprises a DNA fragment encoding a 5' untranslated leader (5'UTL) sequence of an RNA located between the transcription start site and the translation start site. It is believed that the 5' transcription start site of the GL4 promoter is located around position 1197 in SEQ ID No 2, resulting in a 5'UTL of about 30 nucleotides in length. It is believed that the 5' transcription start site of the GL5 promoter is located around position 1280 in SEQ ID No 14, resulting in a 5'UTL of about 30 nucleotides in length. It is also believed that this region can be replaced by another 5'UTL, such as the 5'UTL of another plant-expressible gene, without substantially affecting the specificity of the promoter.

Thus, in another embodiment the invention provides a corn root preferential promoter or corn root preferential promoter region comprising the nucleotide sequence of SEQ ID No 14 from the nucleotide at position 1 to the nucleotide at position 1280, or a nucleotide sequence having at least 95 % sequence identity therewith;

For the purpose of this invention, the "sequence identity" of two related nucleotide or amino acid sequences, expressed as a percentage, refers to the number of positions in the two optimally aligned sequences which have identical residues (x100) divided by the number of positions compared. A gap, i.e. a position in an alignment where a residue is present in one sequence but not in the other, is regarded as a position with non-identical residues. The alignment of the two sequences is performed by the Needleman and Wunsch algorithm (Needleman and Wunsch 1970). The computer-assisted sequence alignment above, can be conveniently performed using standard software program such as GAP which is part of the Wisconsin Package Version 10.1 (Genetics Computer Group, Madision, Wisconsin, USA) using the default scoring matrix with a gap creation penalty of 50 and a gap extension penalty of 3.

It goes without saying that promoters and promoter regions of the invention may also comprise additional elements known to improve transcription efficiency such as enhancers, introns, etc.

The invention further includes DNA molecules comprising the corn root preferential promoters of the invention operably linked to one or more heterologous regions coding for a biologically active RNA, peptide or protein. The promoters of the invention may be used to express any heterologous coding region desired.

Thus in another embodiment of the invention, a chimeric gene is provided comprising
a) a corn root preferential promoter region; comprising the nucleotide sequence of SEQ ID No 14 from the nucleotide at position 1 to the nucleotide at position 1280, or a nucleotide sequence having at least 95 % sequences identity therewith;
b) a DNA region of interest, which when transcribed yields a biologically active RNA; and
c) a DNA region comprising a 3' transcription termination and polyadenylation signal functional in plant cells.

The DNA region of interest, or the transcribed RNA may thus encode a protein or polypeptide, but may also encode biologically active RNA, such as an antisense RNA, a sense RNA, or a dsRNA comprising both sense and antisense RNA stretches capable of basepairing and forming a double stranded RNA, as described in WO 99/53050 usable for posttranscriptional gene silencing of a target sequence.

To confer corn rootworm resistance, preferably resistance to Diabrotica barberi, Diabrotica undecimpuncata, Diabrotica virgifera, to plants, such as corn plants, in a root selective or root preferential way, suitable candidate DNA regions to be operably linked to the corn root selective promoters of the invention include the mature VIP1Aa protein when combined with the mature VIP2Aa or VIP2Ab protein of PCT publication WO 96/10083; the corn rootworm toxins of Photorhabdus or Xhenorhabdus spp., e.g., the insecticidal proteins of Photorhabdus luminescens W-14 (Guo et al., 1999, J. Biol. Chem. 274, 9836-9842); the CryET70 protein of WO 00/26378; the insecticidal proteins produced by Bt strains PS80JJ1, PS149B1 and PS167H2 as described in WO 97/40162, particularly the about 14 kD and about 44 kD proteins of Bt strain PS149B1; the Cry3Bb protein of US patent 6,023,013; protease inhibitors such as the N2 and R1 cysteine proteinase inhibitors of soybean (Zhao et al., 1996, Plant Physiol. 111, 1299-1306) or oryzastatine such as rice cystatin (Genbank entry S49967), corn cystatin (Genbank entries D38130, D10622, D63342) such as the corn cystatin expressed in plants as described by Irie et al. (1996, Plant Mol. Biol. 30, 149-157). Also included are all equivalents and variants, such as truncated proteins retaining insecticidal activity, of any of the above proteins.

In one embodiment of the invention, chimeric genes for conferring rootworm resistance in a root preferential way comprise a nucleotide sequence encoding an insecticidal secreted protein (ISP) from Brevibacillus laterosporus, which is insecticidal when ingested by an insect in combination with an ISP complimentary protein, such as another ISP protein, as described in PCT application PCT/EP01/05702 published as WO 01/87931 (particularly DNA sequences SEQ ID- No7 and 9 of WO 01/87931). The nucleotide sequences encoding ISP protein may be a modified DNA.

The invention further provides methods for expressing a foreign DNA of interest preferentially in the roots of a plant, such as a corn plant, comprising the following steps:
a) providing plant cells with the chimeric genes of the invention, preferably stably integrated in their genome, particularly their nuclear genome, to generate transgenic cells;
b) regenerating plants from said transgenic cells.

A convenient way to provide plant cells with the chimeric genes of the invention is to introduce the DNA via conventional transformation methods. It will be clear that actual method of transforming the plants, particularly cereal plants has little importance for the currently described methods and several methods for introduction of foreign DNA into the genome of plant cells including but not limited to, direct protoplast transformation (see e.g. for corn US 5792936) Agrobacterium mediated transformation (see e.g. for corn US 6074877 or US 6140553), microprojectile bombardment, electroporation- of compact embryogenic calli (see e.g. for corn US 5641664) or silicon whisker mediated DNA introduction are available in the art.

Operably linking the foreign DNA of interest to a corn root preferential promoter according to the invention, may also be achieved by replacing the DNA naturally associated with the corn root preferential promoter by homologous recombination with the DNA of interest, provided that said DNA of interest comprises a homology region with the DNA normally associated with the corn root preferential promoter. Methods for introducing DNA of interest into plant cell genome by homologous recombination are available (e.g. US patent 5744336).

The obtained transformed plant can be used in a conventional breeding scheme to produce more transformed plants with the same characteristics or to introduce the chimeric gene for corn root preferential expression according to the invention in other varieties of the same or related plant species, or in hybrid plants. Seeds obtained from the transformed plants contain the chimeric genes of the invention as a stable genomic insert and are also encompassed by the invention.

It will be appreciate that the means and methods of the Invention are particularly useful for corn, but may also be used in other plants with similar effects, particularly in cereal plants including corn, wheat, oat, barley, rye, rice, turfgrass; sorghum, millet or sugarcane plants.

The following Examples describe the isolation of corn root preferential promoters, and the construction of chimeric genes for selective expression in corn root plants. Unless stated otherwise in the Examples, all recombinant DNA techniques are carried out according to standard protocols as described in Sambrook et al. (1989) Molecular Cloning: A Laboratory Manual, Second Edition, Cold Spring Harbor Laboratory Press, NY and in Volumes 1 and 2 of Ausubel et al. (1994) Current Protocols in Molecular Biology, Current Protocols*,* USA. Standard materials and methods for plant molecular work are described in Plant Molecular Biology Labfax (1993) by R.b.D. Croy, jointly published by BIOS Scientific Publications Ltd (UK) and Blackwell Scientific Publications, UK.

Throughout the description and Examples, reference is made to the following sequences represented in the sequence listing:
SEQ ID No 1: nucleotide sequence of the about 400 bp corn root preferential promoter (GL4 promoter)
SEQ ID No 2: nucleotide sequence of the about 1200 bp corn root preferential promoter (GL4 promoter)
SEQ ID No 3: nucleotide sequence of the cDNA of the naturally associated mRNA transcribed under control of the corn root preferential promoter having the nucleotide sequence of SEQ ID No 1 (GL4)
SEQ ID No 4: amino acid sequence of the protein encoded by the cDNA GL4.
SEQ ID No 5: nucleotide sequence of the corn root preferential cDNA GL5.
SEQ ID No 6: amino acid sequence of the protein encoded by the cDNA GL5.
SEQ ID No 7: oligonucleotide primer GVK 27
SEQ ID No 8: oligonucleotide primer GVK28.
SEQ ID No 9: oligonucleotide primer GVK 29.
SEQ ID No 10: oligonucleotide primer GVK 30.
SEQ ID No 11: nucleotide sequence of corn root preferential cDNA GL12
SEQ ID No 12: nucleotide sequence of plasmid pTWV011
SEQ ID No 13: nucleotide sequence of plasmid pTWV018
SEQ ID No 14: nucleotide sequence of the about 1300 bp corn root preferential promoter (GL5 promoter)
SEQ ID No 15: oligonucleotide primer GVK22
SEQ ID No 16: oligonucleotide primer GVK23
SEQ ID No 17: oligonucleotide primer GVK24
SEQ ID No 18: oligonucleotide primer GVK25
SEQ ID No 19: oligonucleotide primer GVK26
SEQ ID No 20: oligonucleotide primer GVK31
SEQ ID No 21: oligonucleotide primer GVK32
SEQ ID No 22: oligonucleotide primer GVK33
SEQ ID No 23: oligonucleotide primer GVK38
SEQ ID No 24: oligonucleotide primer GVK39
SEQ ID No 25: oligonucleotide primer GVK45
SEQ ID No 26: oligonucleotide primer MDB285
SEQ ID No 27: oligonucleotide primer MDB286
SEQ ID No 28: oligonucleotide primer MDB363
SEQ ID No 29: oligonucleotide primer MDB364 .
SEQ ID No 30: oligonucleotide primer MDB552
SEQ ID No 31: oligonucleotide primer MDB556

### EXAMPLES

### Example 1. Isolation of root preferential corn cDNAs

In this reference example, RNA transcript tags which are expressed preferentially in maize roots where identified by a differential RNA cDNA display known as READS (Prashar and Weismann, 1999, Methods in Enzymology 303:258- 272).
To this end, total RNA samples were prepared from different tissues (roots, stems, leaves, ..) of corn plants, harvested at different developmental stages (from 64 day old plants to adult plants). 3' end fragments of cDNA molecules digested by different endorestriction nucleases were amplified using stringent PCR conditions and oligonucleotides as described by Prashar and Weismann (1999, supra) for each of the samples.

Comparison of gel patterns of the 3' end cDNA restriction fragments generated for each of the RNA samples allowed a preliminary identification of fragments which appeared only in the corn root tissue RNA sample or were more prominent in root tissue RNA than in other corn tissue. These 3' end fragments were isolated and sequenced. Their nucleotide sequence was compared against public and proprietary databases and only novel sequences were used in a further Northern analysis using the RNA samples from different corn tissues as a driver and each of the isolated 3' end cDNA fragments as a probe. The hybridizing RNA transcripts were analyzed for size, abundance, and specificity. The results for the 3' end with the best specificity for expression in corn roots are summarized in Table 1, arranged in descending order of specificity and abundance.

**Table 1.**

| Identification of 3' end used as probe | Quantification of hybridizing transcript¹ | Estimated length of hybridizing transcript² | Specificity of presence of the hybridizing transcript |
|---|---|---|---|
| GL4 | 18 | About 600 | Root selective |
| GL5 | 15 | About 600 | Root selective |
| GL12 | 13 | About 600 | Root selective |
| GL11 | 3 | About 820 | Root selective |
| GL3 | 1 | About 1200 | Root selective |
| GL9 | 7 | About 900 | Root preferential |
| GL7 | 2 | About 700 | Root preferential |
| GL16 | <2 | About 1500 | Low expression |
| GL17 | <2 | About 650 | Low expression |
| GL6 | --- | --- | No visible hybridization |

| | | | |
|---|---|---|---|
| ¹expressed in picogram/µg total RNA ²in nucleotide | | | |

The 3' ends which hybridized to the most abundant RNA transcripts with the highest specific expression in corn roots (GL4; GL5 and GL12) were further analyzed.

For GL4 and GL5 full length cDNAs were isolated using the SMART™ RACE cDNA amplification kit from CLONTECH Laboratories with nested oligonucleotide primers GVK22 (SEQ ID No 15)/GVK23 (SEQ ID No 16) for GL4 and GVK24 (SEQ ID No 17)/GVK25 (SEQ ID No 18) for GL5. The nucleotide sequence of the full length cDNAs is represented in SEQ ID NO 3 and 5 respectively. Comparison of both nucleotide sequences revealed that GL4 and GL5 have about 89 % sequence identity.

In both sequences a small ORF could be identified (starting from the nucleotide of SEQ ID NO 3 at position 32 to the nucleotide at position 319 for GL4; the nucleotide of SEQ ID NO 5 at position 27 to the nucleotide at position 307 for GL5). The amino acid sequences of the polypeptides encoded by the ORFs are represented in SEQ ID No 4 or 6. The nucleotide sequence in the region encoding the ORF is more conserved between GAL4 and GL5 cDNA than elsewhere in the fragments.

Using the GL4 cDNA nucleotide sequence as a query, a nucleotide sequences has been identified with 91% sequence identity in the 322 nucleotide overlap (SEQ ID No 19 from WO 00/32799). It has not been described that SEQ ID No 19 from *Zea mays* as described in WO 00/32799 is transcribed in a root selective or root preferential way. Further, a nucleotide sequence (clone MEST23-C07.T3 from a seedling and silk cDNA library) has been identified having 99% sequence identity over 582 nt.

Using the GL5 cDNA nucleotide sequence as a query, a nucleotide sequence has been identified with 85% sequence identity with FtsZ1 related seq from Zea mays (A47325 in Geneseq). It has not been described that this sequence is transcribed in a root selective or root preferential way. Further, a nucleotide sequence (clone MEST523-G12 (3') from a seedling and silk cDNA library) has been identified having 100% sequence identity over 525 nt.

### Example 2. Isolation of corn root preferential promoter regions of the gene transcribing a mRNA, the cDNA of which corresponds to GL4 or GL5 cDNA.

The genomic fragments upstream of the nucleotide sequences corresponding to GL4 cDNA and GL5 cDNA sequences (the former for reference only), comprising the promoter region were isolated using Thermal Asymmetric Interlaced PCR as described by Liu et al. (1995, *The Plant Journal* **8(3)**: 457-463).

Corn genomic DNA for use as the initial template DNA was purified as described by Dellaporte et al. The sequence of the specific nested oligonucleotides used for TAIL-PCR to isolate the genomic fragments located upstream of the genomic DNA sequences corresponding to GL4 cDNA sequences are represented in SEQ ID No 7 (GVK27), SEQ ID No 8 (GVK28) and SEQ ID No 9 (GVK 29) ; the aspecific degenerate primers used were each of the 6 degenerate primers MDB285, MDB286, MDB363, MDB364, MDB552 or MDB556 in separate reactions. PCR conditions were as described in Liu et al (1995, supra).

A genomic fragment of about 400 bp (corresponding to the amplification product obtained with the primer pair (GVK29/MDB285) was isolated, cloned in pGEM-T Easy ® and sequenced.

Based on the nucleotide sequence of the about 400 bp fragment, new specific nested primer oligonucleotides (GVK 31/ SEQ ID No 20; GVK32 / SEQ ID No 21 and GVK33 / SEQ ID No 22) were designed and used in conjunction with the above mentioned degenerated primers to isolate the adjacent DNA regions further upstream of the isolated promoter region fragment. This resulted in isolation of an about 350 nt DNA fragment (corresponding to the amplification product obtained with the primer pair GVK33 / MDB286) In a third round, the adjacent upstream DNA fragment of about 800 nt was isolated using new set of specific nested oligonucleotides GVK33 / SEQ ID No 22; GVK38 / SEQ ID No 23 and GVK39 / SEQ ID No 24 in conjunction with the above mentioned degenerated primers (corresponding to the amplification product using GVK39 and MDB363).

To confirm the continuity of the isolated genomic upstream fragments, the complete 1200 bp DNA fragment was amplified using GVK29 and GVK45 (SEQ ID NO 25) primers and cloned in pGEM-T Easy®. The complete nucleotide sequence of the about 1200 bp upstream DNA fragment is represented in SEQ ID NO 2.

Primers GVK 27, GVK28 and GVK30 (SEQ ID No 10) were used in conjunction with the above mentioned degenerated primers. An about 1300 nt fragment was amplified having the sequence of SEQ ID NO 14 (corresponding to the amplification product by MDB364 and GVK30).

### Example 3. Construction of chimeric genes using the isolated GL4/GL5 corn root preferential promoter regions.

The following chimeric ISP1A/ISP2A constructs under the control of GL4 promoter region or under control of the GL5 promoter region (the former for reference only) were made using standard recombinant DNA methods:
GL4::ISP1A (for reference only) comprising the following DNA fragments:
   - the GL4 promoter region (SEQ ID No 2)
   - a DNA fragment encoding the isp1A protein of *Brevibacillus laterosporus* (complement of the nucleotide sequence of SEQ ID NO 12 from the nucleotide at position 2003 to the nucleotide at position 4511);
   - the 3' end fragment of the 35S transcript (complement of the nucleotide sequence of SEQ ID No 12 from the nucleotide at position 1767 to the nucleotide at position 1991).
GL4::ISP2A (for reference only) comprising the following DNA fragments:
   - the GL4 promoter region (SEQ ID No 2)
   - a DNA fragment encoding the isp2A protein of *Brevibacillus laterosporus* (complement of the nucleotide sequence of SEQ ID No 12 from the nucleotide at position 6001 to the nucleotide at position 7228);
   - the 3' end fragment of the 35S transcript (complement of the nucleotide sequence of SEQ ID No 12 from the nucleotide at position 5765 to the nucleotide at position 5989).
GLS::ISP1A comprising the following DNA fragments:
   - the GL5 promoter region (complement of the nucleotide sequence of SEQ ID No 13 from the nucleotide at position 4518 to the nucleotide at position 5822)
   - a DNA fragment encoding the isp1A protein of *Brevibacillus laterosporus* (complement of the nucleotide sequence of SEQ ID No 13 from the nucleotide at position 2003 to the nucleotide at position 4511);
   - the 3' end fragment of the 35S transcript (complement of the nucleotide sequence of SEQ ID No 13 from the nucleotide at position 1767 to the nucleotide at position 1991).
GL5::ISP2A comprising the following DNA fragments:
   - the GL5 promoter region (complement of the nucleotide sequence of SEQ. ID No 13 from the nucleotide at position 8628 to the nucleotide at position 7324)
   - a DNA fragment encoding the isp2A protein of *Brevibacillus laterosporus* (complement of the nucleotide sequence of SEQ ID No 12 from the nucleotide at position 6090 to the nucleotide at position 7317);
   - the 3' end fragment of the 35S transcript (complement of the nucleotide sequence of SEQ ID No 12 from the nucleotide at position 5765 to the nucleotide at position 5989).

### Example 4. Expression analysis of chimeric genes comprising the GL4 and GL5 promoters in stably transformed corn plants.

The chimeric genes described in Example 3 were introduced in a T-DNA vector along with a chimeric bar gene (such as described in US patent 5,561,236) to yield pTWV011 (GL4 promoter constructs, for reference only) and pTWV018 (GL5 promoter constructs). These T-DNA vectors were introduced in *Agrobacterium tumefaciens* containing the helper Ti-plasmid pGV4000 or pEHA101.

Corn plants stably transformed with the mentioned chimeric genes were obtained using the Agrobacterium mediated transformation technique described in US 6,140,553.

Corn plants stably transformed with the mentioned chimeric genes may also be obtained using direct DNA delivery to corn protoplast as described in US 5,767,367.

RNA was isolated from root, leaf and stem tissue of these corn plants (grown either in vitro or in vivo) and submitted to Northern analysis using a ISP1A specific probe. The individual results are represented in Table 2.

In summary, after correction for loading by hybridization with ribosomal RNA probe, the GL5 promoter region on average initiated 11 times more transcription in roots than in leaves and 19 times more in roots than in stems. The GL4 promoter region on average initiates 2 times more transcription in roots than in leaves and more than 10 times more in roots than in stems (although individual transformants may exhibit a more pronounced corn root selective expression pattern).

**Table 1. summary of GL4/GL5 transcription data**

| | | **mean** | **SD** | | |
|---|---|---|---|---|---|
| | | n=3 | | | |
| **GI4 promoter** | root | 1.25 | 1.18 | **root/leaf** | **2** |
| | leaf | 0.61 | 0.73 | **root/stem** | **>10** |
| | stem | <0.06 | | | |
| | | | | | |
| **GI5 promoter** | root | 1.32 | 0.24 | **root/leaf** | **11** |
| | leaf | 0.12 | 0.05 | **root/stem** | **19** |
| | stem | 0.07 | 0.03 | | |

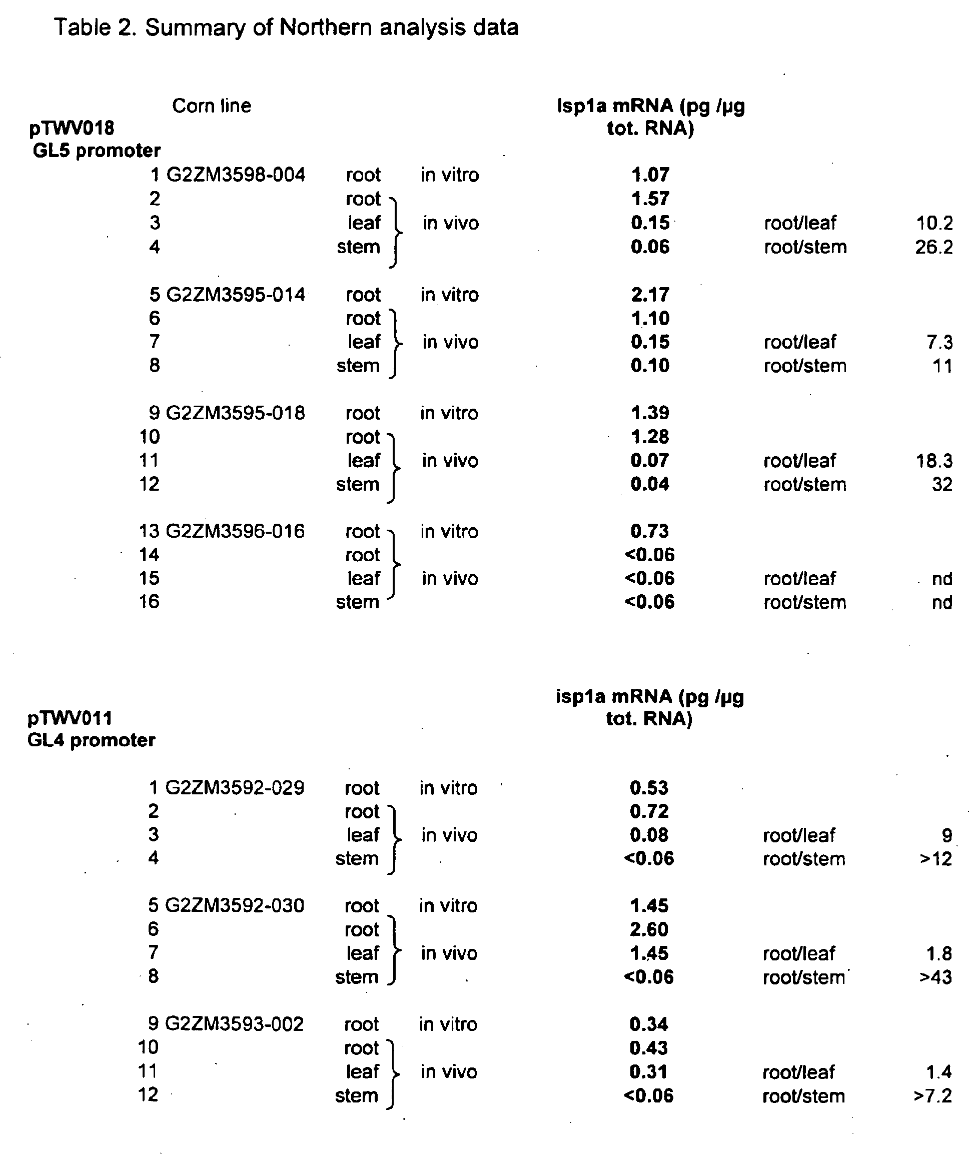

### Example 5. Expression analysis of chimeric genes comprising the GL4 and GL5 promoters in progeny of stably transformed corn plants.

Nine transgenic T0 lines of each of the GL4 and GL5 promoter (the former for reference only) containing corn plants of Example 4 were crossed with untransformed B73, and the T1 plants were analyzed by Southern blot, Northern blot and by ELISA assay for the presence of ISP1 mRNA or protein in various .plant parts.

Southern analysis was performed at the V4 stage to determine the copy number of the transgenes. All analyzed events were single copy events except two lines that contained 2 copies of the transgene.

Northern analysis was performed on RNA derived from root, leaf and stem material obtained at the V11-V13 stage as in Example 4. Transcript levels were quantified using image quant analysis. A correction for loading differences was performed using a ribosomal probe.

For the plants with the GL4 promoter containing transgene, isp1 mRNA was estimated between 0.17 to 0.74 pg/µg total RNA. The average isp1a mRNA level in roots (n=9) was 0.42 pg/µg tot. RNA (SE = 0.18). The average ratio (n=9) of isp1a mRNA in root versus leaf is >6.3. The average ratio (n=8) of isp1a mRNA in root versus stem is >7.1.No expression was seen in stem except for one sample were the ratio root/stem was 1.9.

For the plants with the GL5 promoter containing transgene, isp1 RNA was estimated between 0.95 to 2.55 pg/µg total RNA. The average isp1a mRNA level in roots (n=9) was 1.57 pg/µg tot.RNA (SE = 0.53). The average ratio (n=9) of isp1a mRNA in root versus leaf is >17.6. The average ratio (n=8) of isp1a mRNA in root versus stem is >26.6. No expression was observed in stem.

Root, leaf and stem material, harvested at the V8 stage, was analyzed at the protein level for the presence of ISP1a protein by ELISA. Two plants per event were analyzed. As a negative control, root leaf and stem for wtB73 was checked for ISP1A protein. No ISP1A protein was detected in the control experiments.

For all events no ISP1A protein expression was detected in leaves or stem. The mean value of levels of ISP1A protein detected in roots were:
- 0.07 pg/ml corresponding to about 0.024% of total protein level (n=18) for roots of plants containing the GL4 promoter driven transgene.
- 0.12 pg/ml corresponding to about 0.041% of total protein level (n=18) for roots of plants containing the GL5 promoter driven transgene.

Root, leaf, stem and pollen material, harvested at the flowering stage, was analyzed at the protein level for the presence of ISP1a protein by ELISA. One plant per event was analyzed. As a negative control, root, leaf, stem and pollen material for wtB73 was checked for ISP1A protein. No ISP1A protein was detected in the control experiments.

For all events, no ISP1A protein expression was detected in pollen.
Mean ISP1A Protein level detected in root, leaf and stem of plants containing the GL4 promoter driven transgene:
mean value (n=9) :
   - root: 0.286µg isp1a/ml~0.116% isp1a of tot. protein level
   - leaf: 0.018µg isp1a/ml ~0.0022% isp1a of tot. protein level (6% of root level)
   - stem: 0.020µg isp1a/ml - 0.0043% isp1a of tot. protein level (7% of root level)
mean ISP1A Protein level detected in root, leaf and stem of plants containing the GL4 promoter driven transgene
mean value (n=9) :
   - root: 0.265µg isp1A/ml ~0.142% isp1a of tot. protein level
   - leaf: 0.013µg isp1A/ml ~0.0015% isp1a of tot. protein level (5% of root level)
   - stem: 0.024µg isp1A/ml ~0.0058% isp1a of tot. protein level (9% of root level)

At seed setting, ISP1A protein level was determined in kernels of the transgenic plants. No ISP1A protein could be detected in seed of the transgenic plants, nor in the wt B73 control.

### SEQUENCE LISTING

<110> Vanderkimpen, Greet
   Van Eldik, Gerben
   Meulewaeter, Frank
<120> Corn root preferential promoters and uses thereof
<130> 1021565-000119
<140> US 10/623,500
   <141> 2003-07-22
<150> US 60/399,983
   <151> 2002-07-31
<160> 31
<170> PatentIn version 3.3
<210> 1
   <211> 378
   <212> DNA
   <213> Zea mays
<220>
   <221> misc_feature
   <222> (1)..(338)
   <223> promoter
<220>
   <221> misc_feature
   <222> (339)..(370)
   <223> 5' UTR
<400> 1
   tactacagat aacacgacag ttaacgagcg ggtatgggtt gttttccttg agcactgttg      60
   ttctctagaa tctctgaatc tctctctgtc ttgatgacac cgagcggaaa tagcagttgg     120
   aagaggtgat tgggcttcag cgcgcgatcc aacccaagtg ggttccacaa cgtgaacctc     180
   atgcagctta aaatacagcc agttgtgatc catctgccac agctgtttct acctcagatg     240
   tgctacacag tgtattacct gtttctacct cgcagatgtg ctacacagtt gcttatgact     300
   gcctataaaa tggccgggat cggtgaggct gctggaacca aggagagaga gcatatatat     360
   ccaccgatcc atggcatg                                                   378
<210> 2
   <211> 1236
   <212> DNA
   <213> Zea mays
<220>
   <221> misc_feature
   <222> (11)..(1196)
   <223> promoter
<220>
   <221> misc_feature
   <222> (1197)..(1228)
   <223> 5' UTR
<400> 2
   cgggatcccg gctttctgca ctggacgtag tgtactttat acttgaaact tgtataaatt 60
   tgtgtctttt atactccctc agtttgaaat atagttcttt ctagcctctt tttttccgtc 120
   cacactcatt tgaatgataa taaatataga tatacataca aactatattc ataggttaat 180
   taataaatgt atatttagtc taaaatgaaa tatattttac ccatcgtatt ccttatgcat 240
   gaaatgttga tctacttgtc tgatggaaaa atactatgac gttgttgtac cagaccgcac 300
   ctaaatcaaa ctgttttcag agatggccat tctattattg tagatttgtg atacgtacga 360
   tgtacttttt tatccataaa ataccgtacc attatgatat ggatatcttg atgagaggga 420
   ctcattatct ctctctatat atataaacac ctatatatca aacaggcatc aagaaaaata 480
   gatgattttt ttttctgaag tagagtgaca gaagcagctg aagtgtgagt ctttttgttt 540
   caattttata atgtgtaaag aaaatgacgc caatgaaata tgtgtctggg ctgacgtgtt 600
   gtttggtgaa agccaatttt gttgtatata ggggggccag agcccagttg tatttgttgc 660
   ccggactggc gccaaaaaaa aaaatccgga tagtactatt ccgctaactg tgtcacactt 720
   tatctaaaat tagtcatcca aattaaagaa ctaaccttag atacaaaaaa ttaaacaaag 780
   tatgacaagt taggtagcaa actaaactaa agaggataac acaacagtta accgtcgacg 840
   tgcgcggcct gaatttacta ctacagataa cacgacagtt aacgagcggg tatgggttgt 900
   tttccttgag cactgttgtt ctctagaatc tctgaatctc tctctgtctt gatgacaccg 960
   agcggaaata gcagttggaa gaggtgattg ggcttcagcg cgcgatccaa cccaagtggg 1020
   ttccacaacg tgaacctcat gcagcttaaa atacagccag ttgtgatcca tctgccacag 1080
   ctgtttctac ctcagatgtg ctacacagtg tattacctgt ttctacctcg cagatgtgct 1140
   acacagttgc ttatgactgc ctataaaatg gccgggatcg gtgaggctgc tggaaccaag 1200
   gagagagagc atatatatcc accgatccat ggcatg 1236
<210> 3
   <211> 592
   <212> DNA
   <213> Artificial
<220>
   <223> cDNA of the GL4 transcript
<220>
   <221> misc feature
   <222> (540)..(540)
   <223> n at position 540 represents any nucleotide
<400> 3
   caaggagaga gagcatatat atccaccgat catgatgaag ggtggcagca agaaggaagt      60
   ggccggtgcg gcggcggtgg tggccatact gctggttctg cagctgatgg cagctccacc     120
   gacggccatg gccgcccgct cgccgcgcgg agccgtgccg gatggctccc tcgccacgac     180
   gcccaaggtg acgatgctgt cagccacgct gtgctacacg ggggagacat gcaaatacat      240
   tacctgcctc actcctgctt gctcctgtaa ctatgatgat cgtcgctgct acatcatatt     300
   tactcctgct gctgcttgag gccattctgt gtacgtgaat gaagccacta ctactctcac     360
   acagcatgcg ccggccgacg acgtgcgtac gtatatatat acgctctacc tcgtgagctt     420
   ttgttcgagt gatacgtgtt tcaaggcatc catccatcca tggatgctta tgtacgtata     480
   tgtgttagtc gtgtgtcagg caaccgggca gcagaagggg gtgttgtatt atatatattn     540
   acgtcttctg gtgattaaat aataaagggg ggcatgttgg atgtgtgcaa aa             592
<210> 4
   <211> 95
   <212> PRT
   <213> Zea mays
<400> 4
   Met Met Lys Gly Gly Ser Lys Lys Glu Val Ala Gly Ala Ala Ala Val
   1               5                   10                  15
   Val Ala Ile Leu Leu Val Leu Gln Leu Met Ala Ala Pro Pro Thr Ala
               20                  25                  30
   Met Ala Ala Arg Ser Pro Arg Gly Ala Val Pro Asp Gly Ser Leu Ala
           35                  40                  45
   Thr Thr Pro Lys Val Thr Met Leu Ser Ala Thr Leu Cys Tyr Thr Gly
       50                  55                  60
   Glu Thr Cys Lys Tyr Ile Thr Cys Leu Thr Pro Ala Cys Ser Cys Asn
   65                  70                  75                  80
   Tyr Asp Asp Arg Arg Cys Tyr Ile Ile Phe Thr Pro Ala Ala Ala
                   85                  90                  95
<210> 5
   <211> 535
   <212> DNA
   <213> Artificial
<220>
   <223> cDNA of the GL5 transcript
<220>
   <221> misc_feature
   <222> (82)..(82)
   <223> n represents any nucleotide
<400> 5
   ccaatcagat agagagcata gtcgatcatg aagggtggca agaagaaagt ggccggtgcg      60
   gtggtggcca tactgctggt tntgcagctc atggcagctc caccgacggc catggccgcc     120
   cgctcgccgc gcggagccgt gccggatggc tccctcgcca cgacgcccaa ggtgacgatg     180
   ctgtcggcca cgctgtgcta cacgggggag acatgcaaat acattggctg cctcactcct     240
   gcttgctcct gcaactatag tgatcgtcta tgctacatca tatttactcc tgttgcttga     300
   ggccattccg cgaagccaca actcttacaa tatgcatgcg ccggccgacg acgacgcgcg     360
   ctgcctctcg tgagcttctg ttcaagtgat gcatgtttca aggcatccat ggatgcttta     420
   cgtatatgcg tattaattag ccgtgtcagg gaaccggaca gaagggggtg ttgttttata     480
   tttacgtctt ctggtgatca aataaagggg aaatatatgt tggatgtgtg caaaa          535
<210> 6
   <211> 90
   <212> PRT
   <213> Zea mays
<400> 6
   Met Lys Gly Gly Lys Lys Glu Val Ala Gly Ala Val Val Ala Ile Leu
   1               5                   10                  15
   Leu Val Leu Gln Leu Met Ala Ala Pro Pro Thr Ala Met Ala Ala Arg
               20                  25                  30
   Ser Pro Arg Gly Ala Val Pro Asp Gly Ser Leu Ala Thr Thr Pro Lys
           35                  40                  45
   Val Thr Met Leu Ser Ala Thr Leu Cys Tyr Thr Gly Glu Thr Cys Lys
       50                  55                  60
   Tyr Ile Gly Cys Leu Thr Pro Ala Cys Ser Cys Asn Tyr Ser Asp Arg
   65                  70                  75                      80
   Leu Cys Tyr Ile Ile Phe Thr Pro Val Ala
                   85                  90
<210> 7
   <211> 24
   <212> DNA
   <213> Artificial
<220>
   <223> oligonucleotide primer GVK27
<400> 7
   gctgacagca tcgtcacctt gggc 24
<210> 8
   <211> 25
   <212> DNA
   <213> Artificial
<220>
   <223> oligonucleotide primer GVK28
<400> 8
   gctgcagaac cagcagtatg gccac 25
<210> 9
   <211> 27
   <212> DNA
   <213> Artificial
<220>
   <223> oligonucleotide primer GVK29
<400> 9
   catgccatgg atcggtggat atatatg 27
<210> 10
   <211> 25
   <212> DNA
   <213> Artificial
<220>
   <223> oligonucelotide primer GVK30
<400> 10
   catgccatgg atcgactatg ctctc 25
<210> 11
   <211> 164
   <212> DNA
   <213> Artificial
<220>
   <223> cDNA of 3' end of the GL12 transcript
<220>
   <221> misc_feature
   <222> (45)..(45)
   <223> n= any nucleotide
<220>
   <221> misc_feature
   <222> (53)..(53)
   <223> n= any nucleotide
<220>
   <221> misc_feature
   <222> (57)..(57)
   <223> n= any nucleotide
<220>
   <221> misc_feature
   <222> (59)..(59)
   <223> n= any nucleotide
<220>
   <221> misc_feature
   <222> (61)..(61)
   <223> n= any nucleotide
<220>
   <221> misc_feature
   <222> (88)..(88)
   <223> n= any nucleotide
<220>
   <221> misc_feature
   <222> (93)..(93)
   <223> n= any nucleotide
<220>
   <221> misc_feature
   <222> (96)..(96)
   <223> n= any nucleotide
<220>
   <221> misc feature
   <222> (126)..(126)
   <223> n= any nucleotide
<220>
   <221> misc feature
   <222> (157)..(157)
   <223> n= any nucleotide
<400> 11
   ttttacacac atccaacatg ccccccttta ttatttaatc accanaagac gtnaatntnt      60
   nttattcaac accccctttt gctgcccngg tgnctnacac accactaaca catttccgtt    120
   cataancatt catgctgggc cgcgctgtgc ccgacanctt aaac                      164
<210> 12
   <211> 8514
   <212> DNA
   <213> Artificial
<220>
   <223> plasmid pTWV011
<220>
   <221> misc_feature
   <222> (52)..(1)
   <223> LB= left T-DNA border
<220>
   <221> misc_feature
   <222> (318)..(58)
   <223> 3' nos
<220>
   <221> misc_feature
   <222> (888)..(337)
   <223> coding region of the bar gene
<220>
   <221> misc_feature
   <222> (1721)..(889)
   <223> 35S promoter
<220>
   <221> misc_feature
   <222> (1991)..(1767)
   <223> 3' end 35S
<220>
   <221> misc_feature
   <222> (4511)..(2003)
   <223> coding region isp1a
<220>
   <221> misc_feature
   <222> (4546)..(9515)
   <223> leader sequence from the corn GL4 transcript
<220>
   <221> misc_feature
   <222> (5732)..(4547)
   <223> corn preferential promoter GL4
<220>
   <221> misc_feature
   <222> (5989)..(5765)
   <223> 3' end 35S
<220>
   <221> misc_feature
   <222> (7228)..(6001)
   <223> isp2a coding region
<220>
   <221> misc_feature
   <222> (7263)..(7232)
   <223> leader sequence of the GL4 transcript
<220>
   <221> misc_feature
   <222> (8949)..(7264)
   <223> corn root preferential promoter GL4
<220>
   <221> misc_feature
   <222> (8514)..(8490)
   <223> RB=right T-DNA border
<400> 12
   cggcaggata tattcaattg taaatggctc catggcgatc gctctagagg atcttcccga     60
   tctagtaaca tagatgacac cgcgcgcgat aatttatcct agtttgcgcg ctatattttg    120
   ttttctatcg cgtattaaat gtataattgc gggactctaa tcataaaaac ccatctcata    180
   aataacgtca tgcattacat gttaattatt acatgcttaa cgtaattcaa cagaaattat    240
   atgataatca tcgcaagacc ggcaacagga ttcaatctta agaaacttta ttgccaaatg    300
   tttgaacgat ctgcttcgga tcctagacgc gtgagatcag atctcggtga cgggcaggac     360
   cggacggggc ggtaccggca ggctgaagtc cagctgccag aaacccacgt catgccagtt     420
   cccgtgcttg aagccggccg cccgcagcat gccgcggggg gcatatccga gcgcctcgtg     480
   catgcgcacg ctcgggtcgt tgggcagccc gatgacagcg accacgctct tgaagccctg     540
   tgcctccagg gacttcagca ggtgggtgta gagcgtggag cccagtcccg tccgctggtg     600
   gcggggggag acgtacacgg tcgactcggc cgtccagtcg taggcgttgc gtgccttcca     660
   ggggcccgcg taggcgatgc cggcgacctc gccgtccacc tcggcgacga gccagggata     720
   gcgctcccgc agacggacga ggtcgtccgt ccactcctgc ggttcctgcg gctcggtacg     780
   gaagttgacc gtgcttgtct cgatgtagtg gttgacgatg gtgcagaccg ccggcatgtc     840
   cgcctcggtg gcacggcgga tgtcggccgg gcgtcgttct gggtccatgg ttatagagag     900
   agagatagat ttatagagag agactggtga tttcagcgtg tcctctccaa atgaaatgaa     960
   cttccttata tagaggaagg gtcttgcgaa ggatagtggg attgtgcgtc atcccttacg    1020
   tcagtggaga tgtcacatca atccacttgc tttgaagacg tggttggaac gtcttctttt    1080
   tccacgatgc tcctcgtggg tgggggtcca tctttgggac cactgtcggc agaggcatct    1140
   tgaatgatag cctttccttt atcgcaatga tggcatttgt aggagccacc ttccttttct    1200
   actgtccttt cgatgaagtg acagatagct gggcaatgga atccgaggag gtttcccgaa    1260
   attatccttt gttgaaaagt ctcaatagcc ctttggtctt ctgagactgt atctttgaca    1320
   tttttggagt agaccagagt gtcgtgctcc accatgttga cgaagatttt cttcttgtca    1380
   ttgagtcgta aaagactctg tatgaactgt tcgccagtct tcacggcgag ttctgttaga    1440
   tcctcgattt gaatcttaga ctccatgcat ggccttagat tcagtaggaa ctaccttttt    1500
   agagactcca atctctatta cttgccttgg tttatgaagc aagccttgaa tcgtccatac    1560
   tggaatagta cttctgatct tgagaaatat gtctttctct gtgttcttga tgcaattagt    1620
   cctgaatctt ttgactgcat ctttaacctt cttgggaagg tatttgatct cctggagatt    1680
   gttactcggg tagatcgtct tgatgagacc tgctgcgtag gaacgcggcc gcgtatacgt    1740
   atcgatatct tcgaattcat atgcatgatc tggattttag tactggattt tggttttagg    1800
   aattagaaat tttattgata gaagtatttt acaaatacaa atacatacta agggtttctt    1860
   atatgctcaa cacatgagcg aaaccctata ggaaccctaa ttcccttatc tgggaactac    1920
   tcacacatta ttatggagaa aatagagaga gatagatttg tagagagaga ctggtgattt    1980
   cagcgtgtcc aagcttgcta gcctcagtcc acagcgaaga tcctcaccac agcggtcctg   2040
   gtggagcccc acaggtcgtc cttgcaggac agggtgtaca ggaaggacct gtcggacttc   2100
   acgttcttga agtccaggtt gatcctgtta gcgttcttgc cggaaccgga gtccagcacg   2160
   gtcttggtct gctcaccgat caccttgtag gtgaaggaga aggtacctgg ggtctgggta   2220
   atcagtgggg agtcgatgaa gtaggtagcg tcgtggtact tcacggccag tgggtcaccg   2280
   aagaagtcga agtcaccctc gatgatgtcg aactgtggca ctgggtcctt gtactcgatc   2340
   tcctcagcac ccacctccac gaaggacacg tcgtcccagt acacgttggt ctggccgtca   2400
   cccttgatgg agatggtgtt gatctcgttg ccctccaggt ttggcaccag gatgttgatc   2460
   ctctggtaac ccacgtggtc cagggtgatg ttgtcggtca cgatggactc ctgcttaccg   2520
   tcgatctcga tgtccacgga caccttggag tcagccttca tgtacaggga cacgtagtag   2580
   tcgatgttct tcttcagctt gttcttggac tcggtggaca gctcggtgaa agcacccttg   2640
   ttagcggacc tgtactgctt cttaccggtg ttaccaccgt tcaccacgta ggtgtagtac   2700
   cagttgccga tcttggtgtt gtcggactca ccaccgtcgt acagggtgga ggtctttatt   2760
   gtgaagttca tcttcggttc gagcttcacg tcgtacaggt tcttcacgtc cttgaaggaa   2820
   ccggtggagt cgttcagctg cttcctgatc tgcttagcgg tgtactcgtc cacgtaggac   2880
   tgcacggaag cctcgaagat cggctggtcg ttgtagaaca gcaggccgtc cttctcctcg   2940
   atctcgtctg ggtaggccag cttcagggcc tccttcacgg ataaatttgg ggtcttgtcc   3000
   tctgggttgg tgtagtcctt agcagccacc ctcttctcgg acatctggtt gccgttgtcg   3060
   acgatgatgc tggcggtctt ggcggagatc tcgtcggtga tgccgttcca gtcaccagcg   3120
   atggtgatgt tgccgttggt gtccttgatg gcgtacttgc cctccacctg gtcggtttcg   3180
   agcaggatcg gcttcttgtt ggacaggtag gtgttcagct gctccttgtt cagtgggatc   3240
   ggcctggagt tgaagtcgtc catcgtgttg atggcgatgc cgttcttgcc cttctctggg   3300
   taggactggt ctggcaggat ggtcagagcg gtggtgttct ccttggcctt gatggtgccg   3360
   atggtggtgc cgtccaggat gaaggaggtg gttggcttgg tctcgtagat agcaccggtg   3420
   cccacgttgt tgtacctcac gttggcgttc aggtaggcgg actcagcacc gttgatgtgg   3480
   gtgccgtcgt tggtggcgga gccccactcg ttggccacgg tctcggagtg ctggtagttc   3540
   acggacacgc cgaaggatgg acccagaccg gaccaaccag cgttcacgtt cacgccctcg   3600
   gtgttggtgt aggaccagtt ggtggactgg gaggactcca cggagtggga caggtcctcg   3660
   ttcttggaca ggatcacctt ttcgagggac acgttcacgg atgggaaagc agccaccagt 3720
   gggttgaagg tctccttggc gttggacagt ggcatgtccc tagcagcctt ctcgtagtcg 3780
   gagtatgggt cgcccacggt gtgggcttcg agtgggttgc tggtgaactt ctggtagccc 3840
   ttggaggcca gggagtcgtc ccacttcacg gccaccttgt tctggatggt gtagccgttc 3900
   tcctcccaca cgtccgggat ggagtcgccg tcggtgtcgg tgtcctcgtc gatgtccctc 3960
   ttggtcttct gggtgaacag gttggtcttg gaggccttct tcaggaacac ctgggtctcc 4020
   ttcttgttga actctgggtt cctcagctcg tcctgctgca cctgctggga gtggttctgg 4080
   gagtcgatct tgaacagctt cagctccttg aagatcttgt tgtcgatgtg cagggcgtcg 4140
   tcggactggt actcgatctt gatctgcacc agctggccct tttcgaggtg cacggactgc 4200
   ttgttgttgc ccttctcgga gatcaccttg ccgtccagct cgatgatggc gttctcgtcg 4260
   tcggacagct tgaaggtgaa gtcaccagtg gcggaggact ggatcaggcc gatccagcgg 4320
   atggagtggt actcctggtg cttctggtcc acgagggtgt tagcggtctg ctggtcgtag 4380
   atcagggtgt tgtccctggt tggagcgaac agggtcaggt cgttgaagtc cttgcccttg 4440
   aagtagtagc ccagcaggcc ctccctgtcg atctggttgt ccttcgaagc ctgggtggtg 4500
   gtggcgatag ccatggatcg gtggatatat atgctctctc tccttggttc cagcagcctc 4560
   accgatcccg gccattttat aggcagtcat aagcaactgt gtagcacatc tgcgaggtag 4620
   aaacaggtaa tacactgtgt agcacatctg aggtagaaac agctgtggca gatggatcac 4680
   aactggctgt attttaagct gcatgaggtt cacgttgtgg aacccacttg ggttggatcg 4740
   cgcgctgaag cccaatcacc tcttccaact gctatttccg ctcggtgtca tcaagacaga 4800
   gagagattca gagattctag agaacaacag tgctcaagga aaacaaccca tacccgctcg 4860
   ttaactgtcg tgttatctgt agtagtaaat tcaggccgcg cacgtcgacg gttaactgtt 4920
   gtgttatcct ctttagttta gtttgctacc taacttgtca tactttgttt aattttttgt 4980
   atctaaggtt agttctttaa tttggatgac taattttaga taaagtgtga cacagttagc 5040
   ggaatagtac tatccggatt tttttttttg gcgccagtcc gggcaacaaa tacaactggg 5100
   ctctggcccc cctatataca acaaaattgg ctttcaccaa acaacacgtc agcccagaca 5160
   catatttcat tggcgtcatt ttctttacac attataaaat tgaaacaaaa agactcacac 5220
   ttcagctgct tctgtcactc tacttcagaa aaaaaaatca tctatttttc ttgatgcctg 5280
   tttgatatat aggtgtttat atatatagag agagataatg agtccctctc atcaagatat 5340
   ccatatcata atggtacggt attttatgga taaaaaagta catcgtacgt atcacaaatc 5400
   tacaataata gaatggccat ctctgaaaac agtttgattt aggtgcggtc tggtacaaca 5460
   acgtcatagt atttttccat cagacaagta gatcaacatt tcatgcataa ggaatacgat 5520
   gggtaaaata tatttcattt tagactaaat atacatttat taattaacct atgaatatag 5580
   tttgtatgta tatctatatt tattatcatt caaatgagtg tggacggaaa aaaagaggct 5640
   agaaagaact atatttcaaa ctgagggagt ataaaagaca caaatttata caagtttcaa 5700
   gtataaagta cactacgtcc agtgcagaaa gccgggatcc ccgggctagg cgcgccatat 5760
   gcatgatctg gattttagta ctggattttg gttttaggaa ttagaaattt tattgataga 5820
   agtattttac aaatacaaat acatactaag ggtttcttat atgctcaaca catgagcgaa 5880
   accctatagg aaccctaatt cccttatctg ggaactactc acacattatt atggagaaaa 5940
   tagagagaga tagatttgta gagagagact ggtgatttca gcgtgtccaa gcttgctagc 6000
   ctcacttggt cagcagggta gcgtccacta cgtacctctt cacacccttg atcaccacct 6060
   cggtgatctt gtcgatgtgg tagttggagt ccttgtcgat caggatctcc ttctcggaag 6120
   cgaagccacc gatagcggac aggtaggcac cagtggaacc ctttggcacc tgcagcctca 6180
   ggatgaactt cctggagccg aaagcggaca gcctctcgga ggacagggag gtggacatgt 6240
   aacccttgtc ctccttcatg gtgttcagga acttctcctc catctccttc agggatggca 6300
   gtgggtcgga aatctggtaa ccgaactcgg ccataccgca ccacctgtac acggtgatgt 6360
   tctctgggat cggctgcttc tccagagcct cggagatgtt cttgatctgg gtgtccagct 6420
   tctcgttgcc ggagccaccc tggttccgca ggtagtcgtt gatctgcttg tagtcctgcc 6480
   tagcgtagcc gtccagggcc tccctctgtg ggtcggtcag gttcttagcc caaccctcgt 6540
   agttcttcat gccccacctg tgagcctcgg cgttgatgtc gttcttgaag tccagggact 6600
   tcttcagggt accctggatc tgcaggcact cgtaaccctt cttcaccacc ttggagatgt 6660
   tgtccacgtg cagcacgtag ccgttgtcga tcagcatctt gtactcgttg ttgttcagga 6720
   tcacaccagc cttggtcggg atggtggagc ccttgccgga tggcacggtc acttggagga 6780
   tgatcctctc cttggaggac acgttctgag cagtcaggtg ggtgtccagg taggagtcga 6840
   acttgatgtc cttgcccagg aactgctcct tgaactgggc ctgcacgtcg gtgttgatgg 6900
   tgttgccctc ggtcagtggc ttgttaaatc cgatggtgga tggctccacg ttcttgtagg 6960
   tcacgatgga ggaggacagg ttggccttgt cgaacatctt gtcgatctcc ttcaggtcct 7020
   tgatctcgtc ctcgaaggag ccagccatgc tgaaggtgat ctccttgtag ttcttcttga   7080
   tgtcgttctt gttgtccagg aaattattca tacgcgtctt ctcggtcacg gtcagcttcc   7140
   actccttctc cttctccttg ccccactcct tggccttctc cttgtcctcc ttgaagtcct   7200
   cggtgttgtt ggtggtcttc actagtgcca tggatcggtg gatatatatg ctctctctcc   7260
   ttggttccag cagcctcacc gatcccggcc attttatagg cagtcataag caactgtgta   7320
   gcacatctgc gaggtagaaa caggtaatac actgtgtagc acatctgagg tagaaacagc   7380
   tgtggcagat ggatcacaac tggctgtatt ttaagctgca tgaggttcac gttgtggaac   7440
   ccacttgggt tggatcgcgc gctgaagccc aatcacctct tccaactgct atttccgctc   7500
   ggtgtcatca agacagagag agattcagag attctagaga acaacagtgc tcaaggaaaa   7560
   caacccatac ccgctcgtta actgtcgtgt tatctgtagt agtaaattca ggccgcgcac   7620
   gtcgacggtt aactgttgtg ttatcctctt tagtttagtt tgctacctaa cttgtcatac   7680
   tttgtttaat tttttgtatc taaggttagt tctttaattt ggatgactaa ttttagataa   7740
   agtgtgacac agttagcgga atagtactat ccggattttt ttttttggcg ccagtccggg   7800
   caacaaatac aactgggctc tggcccccct atatacaaca aaattggctt tcaccaaaca   7860
   acacgtcagc ccagacacat atttcattgg cgtcattttc tttacacatt ataaaattga   7920
   aacaaaaaga ctcacacttc agctgcttct gtcactctac ttcagaaaaa aaaatcatct   7980
   atttttcttg atgcctgttt gatatatagg tgtttatata tatagagaga gataatgagt   8040
   ccctctcatc aagatatcca tatcataatg gtacggtatt ttatggataa aaaagtacat   8100
   cgtacgtatc acaaatctac aataatagaa tggccatctc tgaaaacagt ttgatttagg   8160
   tgcggtctgg tacaacaacg tcatagtatt tttccatcag acaagtagat caacatttca   8220
   tgcataagga atacgatggg taaaatatat ttcattttag actaaatata catttattaa   8280
   ttaacctatg aatatagttt gtatgtatat ctatatttat tatcattcaa atgagtgtgg   8340
   acggaaaaaa agaggctaga aagaactata tttcaaactg agggagtata aaagacacaa   8400
   atttatacaa gtttcaagta taaagtacac tacgtccagt gcagaaagcc gggatccccg   8460
   ggcaggcctg caggtcgacg gccgagtact ggcaggatat ataccgttgt aatt         8514
<210> 13
   <211> 8692
   <212> DNA
   <213> Artificial
<220>
   <223> plasmid pTW018 T-DNA sequence
<220>
   <221> misc_feature
   <222> (25)..(1)
   <223> LB= left border region (complement)
<220>
   <221> misc feature
   <222> (318)..(58)
   <223> 3' nos (complement)
<220>
   <221> misc feature
   <222> (888)..(337)
   <223> coding region of the bar gene (complement)
<220>
   <221> misc_feature
   <222> (1721)..(889)
   <223> 35S promoter (complement)
<220>
   <221> misc feature
   <222> (1991)..(1767)
   <223> 3' 35S (complement)
<220>
   <221> misc feature
   <222> (9511)..(2003)
   <223> coding region ispla (complement)
<220>
   <221> misc_feature
   <222> (9542)..(4518)
   <223> leader sequence from the corn GL5 transcript (complement)
<220>
   <221> misc_feature
   <222> (5630)..(5630)
   <223> n=any nucleotide
<220>
   <221> misc feature
   <222> (5822)..(4543)
   <223> GL5 promoter (complement)
<220>
   <221> misc_feature
   <222> (6078)..(5854)
   <223> 3' 35S (complement)
<220>
   <221> misc_feature
   <222> (7317)..(6090)
   <223> isp2a coding region (complement)
<220>
   <221> misc feature
   <222> (7348)..(7324)
   <223> leader sequence of the corn GL5 transcript (complement)
<220>
   <221> misc feature
   <222> (8436)..(8436)
   <223> n=any nucleotide
<220>
   <221> misc feature
   <222> (8628)..(7349)
   <223> promoter of the corn GL5 transcript (complement)
<220>
   <221> misc feature
   <222> (8692)..(8688)
   <223> Right T-DNA border (complement)
<400> 13
   cggcaggata tattcaattg taaatggctc catggcgatc gctctagagg atcttcccga      60
   tctagtaaca tagatgacac cgcgcgcgat aatttatcct agtttgcgcg ctatattttg     120
   ttttctatcg cgtattaaat gtataattgc gggactctaa tcataaaaac ccatctcata     180
   aataacgtca tgcattacat gttaattatt acatgcttaa cgtaattcaa cagaaattat     240
   atgataatca tcgcaagacc ggcaacagga ttcaatctta agaaacttta ttgccaaatg     300
   tttgaacgat ctgcttcgga tcctagacgc gtgagatcag atctcggtga cgggcaggac     360
   cggacggggc ggtaccggca ggctgaagtc cagctgccag aaacccacgt catgccagtt     420
   cccgtgcttg aagccggccg cccgcagcat gccgcggggg gcatatccga gcgcctcgtg     480
   catgcgcacg ctcgggtcgt tgggcagccc gatgacagcg accacgctct tgaagccctg     540
   tgcctccagg gacttcagca ggtgggtgta gagcgtggag cccagtcccg tccgctggtg     600
   gcggggggag acgtacacgg tcgactcggc cgtccagtcg taggcgttgc gtgccttcca     660
   ggggcccgcg taggcgatgc cggcgacctc gccgtccacc tcggcgacga gccagggata     720
   gcgctcccgc agacggacga ggtcgtccgt ccactcctgc ggttcctgcg gctcggtacg     780
   gaagttgacc gtgcttgtct cgatgtagtg gttgacgatg gtgcagaccg ccggcatgtc     840
   cgcctcggtg gcacggcgga tgtcggccgg gcgtcgttct gggtccatgg ttatagagag     900
   agagatagat ttatagagag agactggtga tttcagcgtg tcctctccaa atgaaatgaa     960
   cttccttata tagaggaagg gtcttgcgaa ggatagtggg attgtgcgtc atcccttacg 1020
   tcagtggaga tgtcacatca atccacttgc tttgaagacg tggttggaac gtcttctttt 1080
   tccacgatgc tcctcgtggg tgggggtcca tctttgggac cactgtcggc agaggcatct 1140
   tgaatgatag cctttccttt atcgcaatga tggcatttgt aggagccacc ttccttttct 1200
   actgtccttt cgatgaagtg acagatagct gggcaatgga atccgaggag gtttcccgaa 1260
   attatccttt gttgaaaagt ctcaatagcc ctttggtctt ctgagactgt atctttgaca 1320
   tttttggagt agaccagagt gtcgtgctcc accatgttga cgaagatttt cttcttgtca 1380
   ttgagtcgta aaagactctg tatgaactgt tcgccagtct tcacggcgag ttctgttaga 1440
   tcctcgattt gaatcttaga ctccatgcat ggccttagat tcagtaggaa ctaccttttt 1500
   agagactcca atctctatta cttgccttgg tttatgaagc aagccttgaa tcgtccatac 1560
   tggaatagta cttctgatct tgagaaatat gtctttctct gtgttcttga tgcaattagt 1620
   cctgaatctt ttgactgcat ctttaacctt cttgggaagg tatttgatct cctggagatt 1680
   gttactcggg tagatcgtct tgatgagacc tgctgcgtag gaacgcggcc gcgtatacgt 1740
   atcgatatct tcgaattcat atgcatgatc tggattttag tactggattt tggttttagg 1800
   aattagaaat tttattgata gaagtatttt acaaatacaa atacatacta agggtttctt 1860
   atatgctcaa cacatgagcg aaaccctata ggaaccctaa ttcccttatc tgggaactac 1920
   tcacacatta ttatggagaa aatagagaga gatagatttg tagagagaga ctggtgattt 1980
   cagcgtgtcc aagcttgcta gcctcagtcc acagcgaaga tcctcaccac agcggtcctg 2040
   gtggagcccc acaggtcgtc cttgcaggac agggtgtaca ggaaggacct gtcggacttc 2100
   acgttcttga agtccaggtt gatcctgtta gcgttcttgc cggaaccgga gtccagcacg 2160
   gtcttggtct gctcaccgat caccttgtag gtgaaggaga aggtacctgg ggtctgggta 2220
   atcagtgggg agtcgatgaa gtaggtagcg tcgtggtact tcacggccag tgggtcaccg 2280
   aagaagtcga agtcaccctc gatgatgtcg aactgtggca ctgggtcctt gtactcgatc 2340
   tcctcagcac ccacctccac gaaggacacg tcgtcccagt acacgttggt ctggccgtca 2400
   cccttgatgg agatggtgtt gatctcgttg ccctccaggt ttggcaccag gatgttgatc 2460
   ctctggtaac ccacgtggtc cagggtgatg ttgtcggtca cgatggactc ctgcttaccg 2520
   tcgatctcga tgtccacgga caccttggag tcagccttca tgtacaggga cacgtagtag 2580
   tcgatgttct tcttcagctt gttcttggac tcggtggaca gctcggtgaa agcacccttg   2640
   ttagcggacc tgtactgctt cttaccggtg ttaccaccgt tcaccacgta ggtgtagtac   2700
   cagttgccga tcttggtgtt gtcggactca ccaccgtcgt acagggtgga ggtctttatt   2760
   gtgaagttca tcttcggttc gagcttcacg tcgtacaggt tcttcacgtc cttgaaggaa   2820
   ccggtggagt cgttcagctg cttcctgatc tgcttagcgg tgtactcgtc cacgtaggac   2880
   tgcacggaag cctcgaagat cggctggtcg ttgtagaaca gcaggccgtc cttctcctcg   2940
   atctcgtctg ggtaggccag cttcagggcc tccttcacgg ataaatttgg ggtcttgtcc   3000
   tctgggttgg tgtagtcctt agcagccacc ctcttctcgg acatctggtt gccgttgtcg   3060
   acgatgatgc tggcggtctt ggcggagatc tcgtcggtga tgccgttcca gtcaccagcg   3120
   atggtgatgt tgccgttggt gtccttgatg gcgtacttgc cctccacctg gtcggtttcg   3180
   agcaggatcg gcttcttgtt ggacaggtag gtgttcagct gctccttgtt cagtgggatc   3240
   ggcctggagt tgaagtcgtc catcgtgttg atggcgatgc cgttcttgcc cttctctggg   3300
   taggactggt ctggcaggat ggtcagagcg gtggtgttct ccttggcctt gatggtgccg   3360
   atggtggtgc cgtccaggat gaaggaggtg gttggcttgg tctcgtagat agcaccggtg   3420
   cccacgttgt tgtacctcac gttggcgttc aggtaggcgg actcagcacc gttgatgtgg   3480
   gtgccgtcgt tggtggcgga gccccactcg ttggccacgg tctcggagtg ctggtagttc   3540
   acggacacgc cgaaggatgg acccagaccg gaccaaccag cgttcacgtt cacgccctcg   3600
   gtgttggtgt aggaccagtt ggtggactgg gaggactcca cggagtggga caggtcctcg   3660
   ttcttggaca ggatcacctt ttcgagggac acgttcacgg atgggaaagc agccaccagt   3720
   gggttgaagg tctccttggc gttggacagt ggcatgtccc tagcagcctt ctcgtagtcg   3780
   gagtatgggt cgcccacggt gtgggcttcg agtgggttgc tggtgaactt ctggtagccc   3840
   ttggaggcca gggagtcgtc ccacttcacg gccaccttgt tctggatggt gtagccgttc   3900
   tcctcccaca cgtccgggat ggagtcgccg tcggtgtcgg tgtcctcgtc gatgtccctc   3960
   ttggtcttct gggtgaacag gttggtcttg gaggccttct tcaggaacac ctgggtctcc   4020
   ttcttgttga actctgggtt cctcagctcg tcctgctgca cctgctggga gtggttctgg   4080
   gagtcgatct tgaacagctt cagctccttg aagatcttgt tgtcgatgtg cagggcgtcg   4140
   tcggactggt actcgatctt gatctgcacc agctggccct tttcgaggtg cacggactgc   4200
   ttgttgttgc ccttctcgga gatcaccttg ccgtccagct cgatgatggc gttctcgtcg   4260
   tcggacagct tgaaggtgaa gtcaccagtg gcggaggact ggatcaggcc gatccagcgg 4320
   atggagtggt actcctggtg cttctggtcc acgagggtgt tagcggtctg ctggtcgtag 4380
   atcagggtgt tgtccctggt tggagcgaac agggtcaggt cgttgaagtc cttgcccttg 4440
   aagtagtagc ccagcaggcc ctccctgtcg atctggttgt ccttcgaagc ctgggtggtg 4500
   gtggcgatag ccatggatcg actatgctct ctatctgatt ggtttggctt tgctccagca 4560
   gccagccatt ttataggcag cagtcactaa actgtaggct gtagcacgtc tgacagacag 4620
   gtagatggat cacaactggc tgtattttaa aaagctgcac gaggttcacg ttgtgtcgtc 4680
   gtggtataga taaatgtgca tgcagcaatg gaacaatatt ggggttgatg actgaatcgc 4740
   tcaagctagc tagcccaatc atctcttcca actgctaccc gctgtgtctc ataaacacgc 4800
   aggtccagcg attctaaacc gcaacagtgc tcaacgaaaa ctacccttac ccgctggtta 4860
   attattgtgt tatcatattt aaatgctgtc attttcttta caaattataa aacttgggac 4920
   gtgtttggtt cgctgcctat acttatttta tgtattggat tctatgcgca aagagcaaaa 4980
   ttccagtacc aaatgtttgt tgtattttat tgggtagcgt gtacgtcgca cattctgtaa 5040
   tacaacctcc gttcacagat atatgacatg ttgatttttt taaaaaactt tgaccattta 5100
   tcttattcaa aagtataaaa ttttaattaa gcacaaacta ccttaagtga taaaacaaac 5160
   cacacaaaaa ataaatgaca actcattatt ttttaaataa gacaagtgat taaagttttt 5220
   taaaaagtca gcgatgtcat atatttatga acggtatata tatatatata tataacaccc 5280
   atatcgagca ggcatcaaga aaaacatatc gatgattttt gttttcctaa agtagagtga 5340
   caagctaaac aaatgacata tttttgtttc agttttgtaa ggccattctc agtggtgagc 5400
   ttcagaacat gtgacatatt ttttttgttt tagttttgta aggcctttct cagtggtgag 5460
   ctttagaaca agtgacatct ttttgtttca gttttgtaag gtcattctta gtggtgagct 5520
   tcagaacaag tgagatgaga tctttttgtt tcagttttgt taggccagtt tcatcggtga 5580
   gcttcacaac aagtgacatc ttcttttcag ttttgtaagg ccattttcan cggtgagctt 5640
   cggtacaatg ttttccatgt tgtcacacca tatttaaact aggtaattgt atatatagaa 5700
   ttttatctct atgaaactct accatctccc ataagctctt tctatatctc tgcttttaat 5760
   tgtatgtcat gtcactatgt atgatggtgt atcatcgtat ataatgagta tgaaattccg 5820
   ccaatcacta ggggctaggc gcgccatatg catgatctgg attttagtac tggattttgg 5880
   ttttaggaat tagaaatttt attgatagaa gtattttaca aatacaaata catactaagg 5940
   gtttcttata tgctcaacac atgagcgaaa ccctatagga accctaattc ccttatctgg 6000
   gaactactca cacattatta tggagaaaat agagagagat agatttgtag agagagactg 6060
   gtgatttcag cgtgtccaag cttgctagcc tcacttggtc agcagggtag cgtccactac 6120
   gtacctcttc acacccttga tcaccacctc ggtgatcttg tcgatgtggt agttggagtc 6180
   cttgtcgatc aggatctcct tctcggaagc gaagccaccg atagcggaca ggtaggcacc 6240
   agtggaaccc tttggcacct gcagcctcag gatgaacttc ctggagccga aagcggacag 6300
   cctctcggag gacagggagg tggacatgta acccttgtcc tccttcatgg tgttcaggaa 6360
   cttctcctcc atctccttca gggatggcag tgggtcggaa atctggtaac cgaactcggc 6420
   cataccgcac cacctgtaca cggtgatgtt ctctgggatc ggctgcttct ccagagcctc 6480
   ggagatgttc ttgatctggg tgtccagctt ctcgttgccg gagccaccct ggttccgcag 6540
   gtagtcgttg atctgcttgt agtcctgcct agcgtagccg tccagggcct ccctctgtgg 6600
   gtcggtcagg ttcttagccc aaccctcgta gttcttcatg ccccacctgt gagcctcggc 6660
   gttgatgtcg ttcttgaagt ccagggactt cttcagggta ccctggatct gcaggcactc 6720
   gtaacccttc ttcaccacct tggagatgtt gtccacgtgc agcacgtagc cgttgtcgat 6780
   cagcatcttg tactcgttgt tgttcaggat cacaccagcc ttggtcggga tggtggagcc 6840
   cttgccggat ggcacggtca cttggaggat gatcctctcc ttggaggaca cgttctgagc 6900
   agtcaggtgg gtgtccaggt aggagtcgaa cttgatgtcc ttgcccagga actgctcctt 6960
   gaactgggcc tgcacgtcgg tgttgatggt gttgccctcg gtcagtggct tgttaaatcc 7020
   gatggtggat ggctccacgt tcttgtaggt cacgatggag gaggacaggt tggccttgtc 7080
   gaacatcttg tcgatctcct tcaggtcctt gatctcgtcc tcgaaggagc cagccatgct 7140
   gaaggtgatc tccttgtagt tcttcttgat gtcgttcttg ttgtccagga aattattcat 7200
   acgcgtcttc tcggtcacgg tcagcttcca ctccttctcc ttctccttgc cccactcctt 7260
   ggccttctcc ttgtcctcct tgaagtcctc ggtgttgttg gtggtcttca ctagtgccat 7320
   ggatcgacta tgctctctat ctgattggtt tggctttgct ccagcagcca gccattttat 7380
   aggcagcagt cactaaactg taggctgtag cacgtctgac agacaggtag atggatcaca 7440
   actggctgta ttttaaaaag ctgcacgagg ttcacgttgt gtcgtcgtgg tatagataaa 7500
   tgtgcatgca gcaatggaac aatattgggg ttgatgactg aatcgctcaa gctagctagc 7560
   ccaatcatct cttccaactg ctacccgctg tgtctcataa acacgcaggt ccagcgattc 7620
   taaaccgcaa cagtgctcaa cgaaaactac ccttacccgc tggttaatta ttgtgttatc 7680
   atatttaaat gctgtcattt tctttacaaa ttataaaact tgggacgtgt ttggttcgct 7740
   gcctatactt attttatgta ttggattcta tgcgcaaaga gcaaaattcc agtaccaaat 7800
   gtttgttgta ttttattggg tagcgtgtac gtcgcacatt ctgtaataca acctccgttc 7860
   acagatatat gacatgttga tttttttaaa aaactttgac catttatctt attcaaaagt 7920
   ataaaatttt aattaagcac aaactacctt aagtgataaa acaaaccaca caaaaaataa 7980
   atgacaactc attatttttt aaataagaca agtgattaaa gttttttaaa aagtcagcga 8040
   tgtcatatat ttatgaacgg tatatatata tatatatata acacccatat cgagcaggca 8100
   tcaagaaaaa catatcgatg atttttgttt tcctaaagta gagtgacaag ctaaacaaat 8160
   gacatatttt tgtttcagtt ttgtaaggcc attctcagtg gtgagcttca gaacatgtga 8220
   catatttttt ttgttttagt tttgtaaggc ctttctcagt ggtgagcttt agaacaagtg 8280
   acatcttttt gtttcagttt tgtaaggtca ttcttagtgg tgagcttcag aacaagtgag 8340
   atgagatctt tttgtttcag ttttgttagg ccagtttcat cggtgagctt cacaacaagt 8400
   gacatcttct tttcagtttt gtaaggccat tttcancggt gagcttcggt acaatgtttt 8460
   ccatgttgtc acaccatatt taaactaggt aattgtatat atagaatttt atctctatga 8520
   aactctacca tctcccataa gctctttcta tatctctgct tttaattgta tgtcatgtca 8580
   ctatgtatga tggtgtatca tcgtatataa tgagtatgaa attccgccaa tcactagggg 8640
   caggcctgca ggtcgacggc cgagtactgg caggatatat accgttgtaa tt 8692
<210> 14
   <211> 1316
   <212> DNA
   <213> Zea mays
<400> 14
   ggcggaattt catactcatt atatacgatg atacaccatc atacatagtg acatgacata 60
   caattaaaag cagagatata gaaagagctt atgggagatg gtagagtttc atagagataa 120
   aattctatat atacaattac ctagtttaaa tatggtgtga caacatggaa aacattgtac 180
   cgaagctcac cgctgaaaat ggccttacaa aactgaaaag aagatgtcac ttgttgtgaa 240
   gctcaccgat gaaactggcc taacaaaact gaaacaaaaa gatctcatct cacttgttct 300
   gaagctcacc actaagaatg accttacaaa actgaaacaa aaagatgtca cttgttctaa 360
   agctcaccac tgagaaaggc cttacaaaac taaaacaaaa aaaatatgtc acatgttctg     420
   aagctcacca ctgagaatgg ccttacaaaa ctgaaacaaa aatatgtcat ttgtttagct     480
   tgtcactcta ctttaggaaa acaaaaatca tcgatatgtt tttcttgatg cctgctcgat     540
   atgggtgtta tatatatata tatatatacc gttcataaat atatgacatc gctgactttt     600
   taaaaaactt taatcacttg tcttatttaa aaaataatga gttgtcattt attttttgtg    660
   tggtttgttt tatcacttaa ggtagtttgt gcttaattaa aattttatac ttttgaataa     720
   gataaatggt caaagttttt taaaaaaatc aacatgtcat atatctgtga acggaggttg     780
   tattacagaa tgtgcgacgt acacgctacc caataaaata caacaaacat ttggtactgg     840
   aattttgctc tttgcgcata gaatccaata cataaaataa gtataggcag cgaaccaaac     900
   acgtcccaag ttttataatt tgtaaagaaa atgacagcat ttaaatatga taacacaata     960
   attaaccagc gggtaagggt agttttcgtt gagcactgtt gcggtttaga atcgctggac    1020
   ctgcgtgttt atgagacaca gcgggtagca gttggaagag atgattgggc tagctagctt    1080
   gagcgattca gtcatcaacc ccaatattgt tccattgctg catgcacatt tatctatacc    1140
   acgacgacac aacgtgaacc tcgtgcagct ttttaaaata cagccagttg tgatccatct    1200
   acctgtctgt cagacgtgct acagcctaca gtttagtgac tgctgcctat aaaatggctg    1260
   gctgctggag caaagccaaa ccaatcagat agagagcata gtcgatccat ggcatg        1316
<210> 15
   <211> 25
   <212> DNA
   <213> Artificial
<220>
   <223> oligonucleotide primer GVK22
<400> 15
   gctgtgtgag agtagtagtg gcttc 25
<210> 16
   <211> 24
   <212> DNA
   <213> Artificial
<220>
   <223> oligonucleotide primer GVK23
<400> 16
   cacaagcgtg gctgacagca tcgt 24
<210> 17
   <211> 26
   <212> DNA
   <213> Artificial
<220>
   <223> oligonucleotide primer GVK24
<400> 17
   gctcacgaga ggcagcgcgc gtcgtc 26
<210> 18
   <211> 21
   <212> DNA
   <213> Artificial
<220>
   <223> oligonucleotide primer GVK25
<400> 18
   gtaaaagttg tggcttcccg g 21
<210> 19
   <211> 25
   <212> DNA
   <213> Artificial
<220>
   <223> oligonucleotide primer GVK26
<400> 19
   ccgcgctgtg cccgacagct taaac 25
<210> 20
   <211> 27
   <212> DNA
   <213> Artificial
<220>
   <223> oligonucleotide primer GVK31
<400> 20
   gggttggatc gcgccaatca cctcttc 27
<210> 21
   <211> 24
   <212> DNA
   <213> Artificial
<220>
   <223> oligonucleotide primer GVK32
<400> 21
   ccgctcggtg tcatcaagac agag 24
<210> 22
   <211> 26
   <212> DNA
   <213> Artificial
<220>
   <223> oligonucleotide primer GVK33
<400> 22
   gctcaaggaa aacaacccat acccgc 26
<210> 23
   <211> 24
   <212> DNA
   <213> Artificial
<220>
   <223> oligonucleotide primer GVK38
<400> 23
   ggcgccagtc cgggcaacaa atac 24
<210> 24
   <211> 25
   <212> DNA
   <213> Artificial
<220>
   <223> oligonucleotide primer GVK39
<400> 24
   gggctctggc ccccctatat acaac 25
<210> 25
   <211> 27
   <212> DNA
   <213> Artificial
<220>
   <223> oligonucleotide primer GVK45
<400> 25
   cgggatcccg gctttctgca ctggacg 27
<210> 26
   <211> 15
   <212> DNA
   <213> Artificial
<220>
   <223> oligonucleotide primer MDB285
<220>
   <221> misc_feature
   <222> (1).. (1)
   <223> n=any nucleotide
<400> 26
   ntcgastwts gwgtt 15
<210> 27
   <211> 16
   <212> DNA
   <213> Artificial
<220>
   <223> oligonucleotide primer MDB286
<220>
   <221> misc_feature
   <222> (1)..(1)
   <223> n=any nucleotide
<220>
   <221> misc_feature
   <222> (11)..(11)
   <223> n=any nucleotide
<400> 27
   ngtcgaswga nawgaa 16
<210> 28
   <211> 15
   <212> DNA
   <213> Artificial
<220>
   <223> oligonucleotide primer MDB363
<220>
   <221> misc_feature
   <222> (4)..(4)
   <223> n is a, c, g, or t
<220>
   <221> misc_feature
   <222> (5)..(5)
   <223> n=any nucleotide
<220>
   <221> misc_feature
   <222> (9)..(9)
   <223> n is a, c, g, or t
<220>
   <221> misc_feature
   <222> (10)..(10)
   <223> n=any nucleotide
<400> 28
   sggntgawnt aawac 15
<210> 29
   <211> 15
   <212> DNA
   <213> Artificial
<220>
   <223> oligonucleotide primer MDB364
<220>
   <221> misc_feature
   <222> (5)..(5)
   <223> n=any nucleotide
<400> 29
   sscgnaawtt catwc 15
<210> 30
   <211> 15
   <212> DNA
   <213> Artificial
<220>
   <223> oligonucleotide primer MDB552
<220>
   <221> misc_feature
   <222> (1)..(1)
   <223> n=any nucleotide
<400> 30
   ngtcsagwaw scatt 15
<210> 31
   <211> 16
   <212> DNA
   <213> Artificial
<220>
   <223> oligonucleotide primer MDB556
<220>
   <221> misc_feature
   <222> (2)..(2)
   <223> n=any nucleotide
<220>
   <221> misc_feature
   <222> (6)..(6)
   <223> n=any nucleotide
<400> 31
   cngasnagwt wgcata 16

## Claims

1. A corn root preferential promoter fragment comprising the nucleotide sequence of SEQ ID No 14 from the nucleotide at position 1 to the nucleotide at position 1280 or a nucleotide sequence having at least 95% sequence identity therewith.

2. A corn root preferential promoter region comprising a corn root preferential promoter according to claim 1.

3. The corn root preferential promoter region according to claim 2, further comprising the nucleotide sequence of SEQ ID No 14 from the nucleotide at position 1281 to the nucleotide at position 1308.

4. A corn root preferential promoter region according to claim 2, further comprising the nucleotide sequence of SEQ ID No 1 from the nucleotide at position 339 to the nucleotide at position 366.

5. A chimeric gene comprising the following operably linked DNA regions
a) a corn root preferential promoter according to claim 1 ;
b) a heterologous DNA region encoding a biologically active RNA of interest; and
c) a transcription termination and polyadenylation signal.

6. The chimeric gene according to claim 5, wherein said biologically active RNA encodes a protein of interest.

7. The chimeric gene according to claim 6, wherein said protein is a protein which when expressed in the cells of a plant confers pest or pathogen resistance to said plant.

8. The chimeric gene according to claim 7, wherein said protein is ISP1A or ISP2A from *Brevibacillus laterosporus.*

9. A plant cell comprising a chimeric gene according to any one of claims 5 to 8.

10. A plant comprising in its cells a chimeric gene according to any one of claims 5 to 8.

11. The plant according to claim 10, which is a corn plant.

12. A seed of a plant comprising in its cells a chimeric gene according to any one of claims 5 to 8.

13. A method for expressing a biologically active RNA preferentially in the roots of a plant, said method comprising
a) providing the cells of the roots of said plants with a chimeric gene according to any one of claims 5 to 8; and
b) growing said plants.

14. The method according to claim 13, wherein said plant is a corn plant.

15. Use of a corn root preferential promoter of claim 1 for preferential expression of a biologically active RNA in roots of a plant.

16. The use according to claim 15, wherein said plant is a corn plant.

## Patentansprüche

1. Promoterfragment mit Bevorzugung für die Maiswurzel, das die Nukleotidsequenz SEQ ID Nr. 14 von dem Nukleotid in Position 1 bis zu dem Nukleotid in Position 1280 oder eine Nukleotidsequenz mit mindestens 95% Sequenzidentität dazu umfaßt.

2. Promoterregion mit Bevorzugung für die Maiswurzel, die einen Promoter mit Bevorzugung für die Maiswurzel nach Anspruch 1 umfaßt.

3. Promoterregion mit Bevorzugung für die Maiswurzel nach Anspruch 2, die weiterhin die Nukleotidsequenz SEQ ID Nr. 14 von dem Nukleotid in Position 1281 bis zu dem Nukleotid in Position 1308 umfaßt.

4. Promoterregion mit Bevorzugung für die Maiswurzel nach Anspruch 2, die weiterhin die Nukleotidsequenz SEQ ID Nr. 1 von dem Nukleotid in Position 339 bis zu dem Nukleotid in Position 366 umfaßt.

5. Chimäres Gen, das die folgenden operativ verknüpften DNA-Regionen umfaßt:
a) einen Promoter mit Bevorzugung für die Maiswurzel nach Anspruch 1;
b) eine heterologe DNA-Region, die für eine interessierende biologisch aktive RNA codiert; und
c) ein Transkriptionsterminations- und Polyadenylationssignal.

6. Chimäres Gen nach Anspruch 5, wobei die biologisch aktive RNA für ein interessierendes Protein codiert.

7. Chimäres Gen nach Anspruch 6, wobei es sich bei dem Protein um ein Protein handelt, das, wenn es in den Zellen exprimiert wird, dieser Pflanze Schädlings- oder Pathogenresistenz verleiht.

8. Chimäres Gen nach Anspruch 7, wobei es sich bei dem Protein um ISP1A oder ISP2A aus Brevibacillus laterosporus handelt.

9. Pflanzenzelle, die ein chimäres Gen nach einem der Ansprüche 5 bis 8 umfaßt

10. Pflanze, die in ihren Zellen ein chimäres Gen nach einem der Ansprüche 5 bis 8 umfaßt.

11. Pflanze nach Anspruch 10, bei der es sich um eine Maispflanze handelt.

12. Samen einer Pflanze, die in ihren zellen ein chimäres Gen nach einem der Ansprüche 5 bis 8 umfaßt.

13. Verfahren zum Exprimieren einer biologisch aktiven RNA, vorzugsweise in den Wurzeln einer Pflanze, wobei das Verfahren folgendes umfaßt:
a) Bereitstellen eines chimären Gens nach einem der Ansprüche 5 bis 8 an die Zellen der Wurzeln dieser Pflanze; und
b) Heranziehen dieser Pflanzen.

14. Verfahren nach Anspruch 13, wobei es sich bei der Pflanze um eine Maispflanze handelt.

15. Verwendung eines Promoters mit Bevorzugung für die Maiswurzel nach Anspruch 1 für die bevorzugte Expression einer biologisch aktiven RNA in Wurzeln einer Pflanze.

16. Verwendung nach Anspruch 15, wobei es sich bei der Pflanze um eine Maispflanze handelt.

## Revendications

1. Fragment de promoteur d'expression préférentielle dans les racines de maïs, comprenant la séquence nucléotidique de SEQ ID NO : 14 du nucléotide à la position 1 au nucléotide à la position 1280, ou une séquence nucléotidique ayant au moins 95 % d'identité de séquence avec celles-ci.

2. Région de promoteur d'expression préférentielle dans les racines de maïs comprenant un promoteur d'expression préférentielle dans les racines de maïs selon la revendication 1.

3. Région de promoteur d'expression préférentielle dans les racines de maïs selon la revendication 2, comprenant en outre la séquence nucléotidique de SEQ ID NO : 14 du nucléotide à la position 1281 au nucléotide à la position 1308.

4. Région de promoteur d'expression préférentielle dans les racines de maïs selon la revendication 2, comprenant en outre la séquence nucléotidique de SEQ ID NO : 1 du nucléotide à la position 339 au nucléotide à la position 366.

5. Gène chimère comprenant les régions d'ADN suivantes liées de manière opérationnelle :
a) un promoteur d'expression préférentielle dans les racines de maïs selon la revendication 1 ;
b) une région d'ADN hétérologue codant pour un ARN biologiquement actif d'intérêt ;
et
c) un signal de terminaison de la transcription et de polyadénylation.

6. Gène chimère selon la revendication 5, **caractérisé en ce que** ledit ARN biologiquement actif code pour une protéine d'intérêt.

7. Gène chimère selon la revendication 6, **caractérisé en ce que** ladite protéine est une protéine qui, lorsqu'elle est exprimée dans les cellules d'une plante, confère une résistance vis-à-vis d'organismes nuisibles ou de pathogènes à ladite plante.

8. Gène chimère selon la revendication 7, **caractérisé en ce que** ladite protéine est ISP1A ou ISP2A de Brevibacillus laterosporus.

9. Cellule végétale comprenant un gène chimère selon l'une quelconque des revendications 5 à 8.

10. Plante comprenant dans ses cellules un gène chimère selon l'une quelconque des revendications 5 à 8.

11. Plante selon la revendication 10, qui est une plante de maïs.

12. Graine d'une plante comprenant dans ses cellules un gène chimère selon l'une quelconque des revendications 5 à 8.

13. Procédé d'expression préférentielle d'un ARN biologiquement actif dans les racines d'une plante, ledit procédé comprenant les étapes consistant à :
a) procurer aux cellules des racines desdites plantes un gène chimère selon l'une quelconque des revendications 5 à 8 ; et
b) cultiver lesdites plantes.

14. Procédé selon la revendication 13, **caractérisé en ce que** ladite plante est une plante de maïs.

15. Utilisation d'un promoteur d'expression préférentielle dans les racines de maïs selon la revendication 1, pour l'expression préférentielle d'un ARN biologiquement actif dans les racines d'une plante.

16. Utilisation selon la revendication 15, **caractérisée en ce que** ladite plante est une plante de maïs.
